# EUROPEAN PATENT APPLICATION

(11) **EP 3 243 907 A1**
(43) Date of publication of application: **15.11.2017**
(21) Application number: 16169695.0
(22) Date of filing: 13.05.2016
(51) Int. Cl.: C12Q 1/68

(54) **STABLE PAN-GENOMES AND THEIR USE**

(71) Applicant: Curetis GmbH, 71088 Holzgerlingen (DE)
(72) Inventor: FRÖSE, Patrick, 80539 München (DE); POSCH, Andreas, Emanuel, 1090 Wien (AT); GALATA, Valentina, 66123 Saarbrücken (DE); KELLER, Andreas, 66346 Püttlingen (DE)
(74) Representative: Schnappauf, Georg

(57) **Abstract**

The present invention relates to pan-genomes of bacterial species that are stable with regard to their core genome and the addition of new genes, their uses, as well as a data base containing these pan-genomes, a method of determining an antimicrobial drug profile using a pan-genome, a method of determining an infection of a patient and a method of selecting a treatment of a patient using a pan-genome, and a computer program product for carrying out the methods.

## Description

The present invention relates to pan-genomes of bacterial species that are stable with regard to their core genome and the addition of new genes, their uses, as well as a data base containing these pan-genomes, a method of determining an antimicrobial drug profile using a pan-genome, a method of determining an infection of a patient and a method of selecting a treatment of a patient using a pan-genome, and a computer program product for carrying out the methods.

Antibiotic resistance is a form of drug resistance whereby a sub-population of a microorganism, e.g. a strain of a bacterial species, can survive and multiply despite exposure to an antibiotic drug. It is a serious health concern for the individual patient as well as a major public health issue. Timely treatment of a bacterial infection requires the analysis of clinical isolates obtained from patients with regard to antibiotic resistance, in order to select an efficacious therapy. Generally, for this purpose an association of the identified resistance with a certain microorganism (i.e. ID) is necessary.

Antibacterial drug resistance (ADR) represents a major health burden. The presence and genesis of bacterial resistance against active agents is more rapidly gaining importance as assumed. The increased usage of available drugs leads to multi-resistant bacteria, which in turn need even harder medical treatment. According to the World Health Organization's anti-microbial resistance global report on surveillance, ADR leads to 25,000 deaths per year in Europe and 23,000 deaths per year in the US. In Europe, 2.5 million extra hospital days lead to societal cost of 1.5 billion euro. In the US, the direct cost of 2 million illnesses leads to 20 billion dollar direct cost. The overall cost is estimated to be substantially higher, reducing the gross domestic product (GDP) by up to 1.6%.

In general the mechanisms for resistance of bacteria against antimicrobial treatments rely to a very substantial part on the organism's genetics. The respective genes or molecular mechanisms are either encoded in the genome of the bacteria or on plasmids that can be interchanged between different bacteria. The most common resistance mechanisms include:
1) Efflux pumps are high-affinity reverse transport systems located in the membrane that transports the antibiotic out of the cell, e.g. resistance to tetracycline.
2) Specific enzymes modify the antibiotic in a way that it loses its activity. In the case of streptomycin, the antibiotic is chemically modified so that it will no longer bind to the ribosome to block protein synthesis.
3) An enzyme is produced that degrades the antibiotic, thereby inactivating it. For example, the penicillinases are a group of beta-lactamase enzymes that cleave the beta lactam ring of the penicillin molecule.

In addition, some pathogens show natural resistance against drugs. For example, an organism can lack a transport system for an antibiotic or the target of the antibiotic molecule is not present in the organism.

Pathogens that are in principle susceptible to drugs can become resistant by modification of existing genetic material (e.g. spontaneous mutations for antibiotic resistance, happening in a frequency of one in about 100 mio bacteria in an infection) or the acquisition of new genetic material from another source. One example is horizontal gene transfer, a process where genetic material contained in small packets of DNA can be transferred between individual bacteria of the same species or even between different species. Horizontal gene transfer may happen by transduction, transformation or conjugation. Usually, the expression of resistance imparting markers is induced only by presence of a drug.

Generally, testing for susceptibility/resistance to antimicrobial agents is performed by culturing organisms in different concentrations of these agents.

In brief, agar plates are inoculated with patient sample (e.g. urine, sputum, blood, stool) overnight. On the next day individual colonies are used for identification of organisms, either by culturing or using mass spectroscopy. Based on the identity of organisms new plates containing increasing concentration of drugs used for the treatment of these organisms are inoculated and grown for additional 12 - 24 hours. The lowest drug concentration which inhibits growth (minimal inhibitory concentration - MIC) is used to determine susceptibility/resistance for tested drugs. The process takes at least 2 to 3 working days dur-ing which the patient is treated empirically. Automated systems exist from several companies, e.g. Biomeriux (Vitek), Beckman Coulter (Microscan). A significant reduction of time-to-result is needed especially in patients with life-threatening disease and to overcome the widespread misuse of antibiotics.

More recent approaches focus on the genetic constitution of the pathogen and span the whole spectrum from low-plex testing for single resistance markers over small dedicated panels and target enriched sequencing to whole genome sequencing of bacteria. These approaches have demonstrated significant potential to revolutionize care of patients with infectious diseases.

Recent developments include PCR based test kits for fast bacterial identification (e.g. Biomerieux Biofire Tests, Curetis Unyvero Tests). With these test the detection of selected resistance loci is possible for a very limited number of drugs, but no correlation to culture based AST is given. Mass spectroscopy is increasingly used for identification of pathogens in clinical samples (e.g. Bruker Biotyper), and research is ongoing to establish methods for the detection of susceptibility/resistance against antibiotics.

The use of molecular techniques for direct detection of MRSA has become more commonplace especially for screening purposes. Resistance to methicillin is mediated via the mec operon which is part of the staphylococcal cassette chromosome mec (SCCmec). Recently PCR tests were introduced that are based on the detection of the right extremity sequence of the SCCmec in combination with S. aureus specific marker. Initial reports exist that describe culture based susceptibility reports despite detection of the presence of a resistance conferring gene.

It is known that drug resistance can be associated with genetic modifications such as polymorphisms or gene duplications/deletions. This holds for viruses, where resistance testing is established clinical practice (e.g. HIV genotyping). More recently, it has been shown that resistance has also genetic causes in bacteria and even higher organisms, such as humans where tumors resistance against certain cytostatic agents can be linked to genomic mutations.

Wozniak et al. (BMC Genomics 2012, 13(Suppl 7):S23) disclose genetic determinants of drug resistance in Staphylococcus aureus based on genotype and phenotype data. Stoesser et al. disclose prediction of antimicrobial susceptibilities for Escherichia coli and Klebsiella pneumoniae isolates using whole genomic sequence data (J Antimicrob Chemother 2013; 68: 2234-2244).

Chewapreecha et al (Chewapreecha et al (2014) Comprehensive Identification of single nucleotide polymorphisms associated with beta-lactam resistance within pneumococcal mosaic genes. PLoS Genet 10(8): e1004547) used a comparable approach to identify mutations in gram-positive Streptococcus Pneumonia.

However, there is still a need for easy, fast and reliable detection of infections with antimicrobial drug resistant microorganisms, particularly microbial species, and an improved prediction of response to anti-microbial therapy represents still a high unmet clinical need.

### Summary of the invention

The present inventors have found that an antibiotic resistance profile of an unknown microorganism, particularly bacterial microorganism, can be easily obtained using pan-genomes that are stable with regard to their core genes as well as stable with regard to the addition of new genes when adding a further genome of an additional isolate of the microorganism, particularly bacterial microorganism. The present inventors also found further applications of the present pan-genomes, e.g. with regard to the search for a minimum bacterial genome, drug target finding, etc., which can be greatly enhanced using the present stable pan-genomes.

In a first aspect the present invention relates to a data bank comprising at least one pan-genome of one of the following bacterial species: *Escherichia coli, Acinetobacter baumanii, Citrobacter koseri, Enterobacter aerogenes, Enterobacter cloacae, Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas maltophilia,* wherein the pan-genome of *Escherichia coli* comprises or consists of SEQ ID NO 1 - 41296 or comprises or consists of sequences that are at least 90%, preferably at least 95%, particularly preferably at least 99%, identical to SEQ ID NO 1 - 41296 or comprises at least 90%, preferably at least 95%, particularly preferably at least 99%, of SEQ ID NO 1 - 41296, the pan-genome of *Acinetobacter baumanii* comprises or consists of SEQ ID NO 41297 - 71315 or comprises or consists of sequences that are at least 90%, preferably at least 95%, particularly preferably at least 99%, identical to SEQ ID NO 41297 - 71315 or comprises at least 90%, preferably at least 95%, particularly preferably at least 99%, of SEQ ID NO 41297 - 71315, the pan-genome of *Citrobacter koseri* comprises or consists of SEQ ID NO 71316 - 86840 or comprises or consists of sequences that are at least 90%, preferably at least 95%, particularly preferably at least 99%, identical to SEQ ID NO 71316 - 86840 or comprises at least 90%, preferably at least 95%, particularly preferably at least 99%, of SEQ ID NO 71316 - 86840, the pan-genome of *Enterobacter aerogenes* comprises or consists of SEQ ID NO 86841 - 113784 or comprises or consists of sequences that are at least 90%, preferably at least 95%, particularly preferably at least 99%, identical to SEQ ID NO 86841 - 113784 or comprises at least 90%, preferably at least 95%, particularly preferably at least 99%, of SEQ ID NO 86841 - 113784, the pan-genome of *Enterobacter cloacae* comprises or consists of SEQ ID NO 113785 - 171921 or comprises or consists of sequences that are at least 90%, preferably at least 95%, particularly preferably at least 99%, identical to SEQ ID NO 113785 - 171921 or comprises at least 90%, preferably at least 95%, particularly preferably at least 99%, of SEQ ID NO 113785 - 171921, the pan-genome of *Klebsiella oxytoca* comprises or consists of SEQ ID NO 171922 - 209528 or comprises or consists of sequences that are at least 90%, preferably at least 95%, particularly preferably at least 99%, identical to SEQ ID NO 171922 - 209528 or comprises at least 90%, preferably at least 95%, particularly preferably at least 99%, of SEQ ID NO 171922 - 209528, the pan-genome of *Klebsiella pneumoniae* comprises or consists of SEQ ID NO 209529 - 273591 or comprises or consists of sequences that are at least 90%, preferably at least 95%, particularly preferably at least 99%, identical to SEQ ID NO 209529 - 273591 or comprises at least 90%, preferably at least 95%, particularly preferably at least 99%, of SEQ ID NO 209529 - 273591, the pan-genome of *Morganella morganii* comprises or consists of SEQ ID NO 273592 - 293457 or comprises or consists of sequences that are at least 90%, preferably at least 95%, particularly preferably at least 99%, identical to SEQ ID NO 273592 - 293457 or comprises at least 90%, preferably at least 95%, particularly preferably at least 99%, of SEQ ID NO 273592 - 293457, the pan-genome of *Proteus mirabilis* comprises or consists of SEQ ID NO 293458 - 318000 or comprises or consists of sequences that are at least 90%, preferably at least 95%, particularly preferably at least 99%, identical to SEQ ID NO 293458 - 318000 or comprises at least 90%, preferably at least 95%, particularly preferably at least 99%, of SEQ ID NO 293458 - 318000, the pan-genome of *Pseudomonas aeruginosa* comprises or consists of SEQ ID NO 318001 - 368053 or comprises or consists of sequences that are at least 90%, preferably at least 95%, particularly preferably at least 99%, identical to SEQ ID NO 318001 - 368053 or comprises at least 90%, preferably at least 95%, particularly preferably at least 99%, of SEQ ID NO 318001 - 368053, the pan-genome of *Salmonella enterica* comprises or consists of SEQ ID NO 368054 - 404774 or comprises or consists of sequences that are at least 90%, preferably at least 95%, particularly preferably at least 99%, identical to SEQ ID NO 368054 - 404774 or comprises at least 90%, preferably at least 95%, particularly preferably at least 99%, of SEQ ID NO 368054 - 404774, the pan-genome of *Staphylococcus aureus* comprises or consists of SEQ ID NO 404775 - 421990 or comprises or consists of sequences that are at least 90%, preferably at least 95%, particularly preferably at least 99%, identical to SEQ ID NO 404775 - 421990 or comprises at least 90%, preferably at least 95%, particularly preferably at least 99%, of SEQ ID NO 404775 - 421990, the pan-genome of *Serratia marcescens* comprises or consists of SEQ ID NO 421991 - 457642 or comprises or consists of sequences that are at least 90%, preferably at least 95%, particularly preferably at least 99%, identical to SEQ ID NO 421991 - 457642 or comprises at least 90%, preferably at least 95%, particularly preferably at least 99%, of SEQ ID NO 421991 - 457642, the pan-genome of *Shigella boydii* comprises or consists of SEQ ID NO 457643 - 470165 or comprises or consists of sequences that are at least 90%, preferably at least 95%, particularly preferably at least 99%, identical to SEQ ID NO 457643 - 470165 or comprises at least 90%, preferably at least 95%, particularly preferably at least 99%, of SEQ ID NO 457643 - 470165, the pan-genome of *Shigella flexneri* comprises or consists of SEQ ID NO 470166 - 481587 or comprises or consists of sequences that are at least 90%, preferably at least 95%, particularly preferably at least 99%, identical to SEQ ID NO 470166 - 481587 or comprises at least 90%, preferably at least 95%, particularly preferably at least 99%, of SEQ ID NO 470166 - 481587, the pan-genome of *Shigella sonnei* comprises or consists of SEQ ID NO 481588 - 492644 or comprises or consists of sequences that are at least 90%, preferably at least 95%, particularly preferably at least 99%, identical to SEQ ID NO 481588 - 492644 or comprises at least 90%, preferably at least 95%, particularly preferably at least 99%, of SEQ ID NO 481588 - 492644, and the pan-genome of *Stenotrophomonas maltophilia* comprises or consists of SEQ ID NO 492645 - 537429 or comprises or consists of sequences that are at least 90%, preferably at least 95%, particularly preferably at least 99%, identical to SEQ ID NO 492645 - 537429 or comprises at least 90%, preferably at least 95%, particularly preferably at least 99%, of SEQ ID NO 492645 - 537429.

The invention further relates to the use of a data bank of the invention for the determination of bacterial resistance in a sample from a patient.

In another aspect the present invention is directed to a pan-genome of one of the following bacterial species: *Escherichia coli, Acinetobacter baumanii, Citrobacter koseri, Enterobacter aerogenes, Enterobacter cloacae, Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas maltophilia,* wherein the pan-genome of *Escherichia coli* comprises or consists of SEQ ID NO 1 - 41296 or comprises or consists of sequences that are at least 90%, preferably at least 95%, particularly preferably at least 99%, identical to SEQ ID NO 1 - 41296 or comprises at least 90%, preferably at least 95%, particularly preferably at least 99%, of SEQ ID NO 1 - 41296, the pan-genome of *Acinetobacter baumanii* comprises or consists of SEQ ID NO 41297 - 71315 or comprises or consists of sequences that are at least 90%, preferably at least 95%, particularly preferably at least 99%, identical to SEQ ID NO 41297 - 71315 or comprises at least 90%, preferably at least 95%, particularly preferably at least 99%, of SEQ ID NO 41297 - 71315, the pan-genome of *Citrobacter koseri* comprises or consists of SEQ ID NO 71316 - 86840 or comprises or consists of sequences that are at least 90%, preferably at least 95%, particularly preferably at least 99%, identical to SEQ ID NO 71316 - 86840 or comprises at least 90%, preferably at least 95%, particularly preferably at least 99%, of SEQ ID NO 71316 - 86840, the pan-genome of *Enterobacter aerogenes* comprises or consists of SEQ ID NO 86841 - 113784 or comprises or consists of sequences that are at least 90%, preferably at least 95%, particularly preferably at least 99%, identical to SEQ ID NO 86841 - 113784 or comprises at least 90%, preferably at least 95%, particularly preferably at least 99%, of SEQ ID NO 86841 - 113784, the pan-genome of *Enterobacter clo*acae comprises or consists of SEQ ID NO 113785 - 171921 or comprises or consists of sequences that are at least 90%, preferably at least 95%, particularly preferably at least 99%, identical to SEQ ID NO 113785 - 171921 or comprises at least 90%, preferably at least 95%, particularly preferably at least 99%, of SEQ ID NO 113785 - 171921, the pan-genome of *Klebsiella oxytoca* comprises or consists of SEQ ID NO 171922 - 209528 or comprises or consists of sequences that are at least 90%, preferably at least 95%, particularly preferably at least 99%, identical to SEQ ID NO 171922 - 209528 or comprises at least 90%, preferably at least 95%, particularly preferably at least 99%, of SEQ ID NO 171922 - 209528, the pan-genome of *Klebsiella pneumoniae* comprises or consists of SEQ ID NO 209529 - 273591 or comprises or consists of sequences that are at least 90%, preferably at least 95%, particularly preferably at least 99%, identical to SEQ ID NO 209529 - 273591 or comprises at least 90%, preferably at least 95%, particularly preferably at least 99%, of SEQ ID NO 209529 - 273591, the pan-genome of *Morganella morganii* comprises or consists of SEQ ID NO 273592 - 293457 or comprises or consists of sequences that are at least 90%, preferably at least 95%, particularly preferably at least 99%, identical to SEQ ID NO 273592 - 293457 or comprises at least 90%, preferably at least 95%, particularly preferably at least 99%, of SEQ ID NO 273592 - 293457, the pan-genome of *Proteus mirabilis* comprises or consists of SEQ ID NO 293458 - 318000 or comprises or consists of sequences that are at least 90%, preferably at least 95%, particularly preferably at least 99%, identical to SEQ ID NO 293458 - 318000 or comprises at least 90%, preferably at least 95%, particularly preferably at least 99%, of SEQ ID NO 293458 - 318000, the pan-genome of *Pseudomonas aeruginosa* comprises or consists of SEQ ID NO 318001 - 368053 or comprises or consists of sequences that are at least 90%, preferably at least 95%, particularly preferably at least 99%, identical to SEQ ID NO 318001 - 368053 or comprises at least 90%, preferably at least 95%, particularly preferably at least 99%, of SEQ ID NO 318001 - 368053, the pan-genome of *Salmonella enterica* comprises or consists of SEQ ID NO 368054 - 404774 or comprises or consists of sequences that are at least 90%, preferably at least 95%, particularly preferably at least 99%, identical to SEQ ID NO 368054 - 404774 or comprises at least 90%, preferably at least 95%, particularly preferably at least 99%, of SEQ ID NO 368054 - 404774, the pan-genome of *Staphylococcus aureus* comprises or consists of SEQ ID NO 404775 - 421990 or comprises or consists of sequences that are at least 90%, preferably at least 95%, particularly preferably at least 99%, identical to SEQ ID NO 404775 - 421990 or comprises at least 90%, preferably at least 95%, particularly preferably at least 99%, of SEQ ID NO 404775 - 421990, the pan-genome of *Serratia marcescens* comprises or consists of SEQ ID NO 421991 - 457642 or comprises or consists of sequences that are at least 90%, preferably at least 95%, particularly preferably at least 99%, identical to SEQ ID NO 421991 - 457642 or comprises at least 90%, preferably at least 95%, particularly preferably at least 99%, of SEQ ID NO 421991 - 457642, the pan-genome of *Shigella boydii* comprises or consists of SEQ ID NO 457643 - 470165 or comprises or consists of sequences that are at least 90%, preferably at least 95%, particularly preferably at least 99%, identical to SEQ ID NO 457643 - 470165 or comprises at least 90%, preferably at least 95%, particularly preferably at least 99%, of SEQ ID NO 457643 - 470165, the pan-genome of *Shigella flexneri* comprises or consists of SEQ ID NO 470166 - 481587 or comprises or consists of sequences that are at least 90%, preferably at least 95%, particularly preferably at least 99%, identical to SEQ ID NO 470166 - 481587 or comprises at least 90%, preferably at least 95%, particularly preferably at least 99%, of SEQ ID NO 470166 - 481587, the pan-genome of *Shigella sonnei* comprises or consists of SEQ ID NO 481588 - 492644 or comprises or consists of sequences that are at least 90%, preferably at least 95%, particularly preferably at least 99%, identical to SEQ ID NO 481588 - 492644 or comprises at least 90%, preferably at least 95%, particularly preferably at least 99%, of SEQ ID NO 481588 - 492644, and the pan-genome of *Stenotrophomonas maltophilia* comprises or consists of SEQ ID NO 492645 - 537429 or comprises or consists of sequences that are at least 90%, preferably at least 95%, particularly preferably at least 99%, identical to SEQ ID NO 492645 - 537429 or comprises at least 90%, preferably at least 95%, particularly preferably at least 99%, of SEQ ID NO 492645 - 537429.

The present invention also relates to a method of determining an antimicrobial, e.g. antibiotic, drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations by comparison to a first pan-genome to obtain a third data set of genetic variants;
providing a second data set of antimicrobial drug, e.g. antibiotic, resistance and/or susceptibility of the at least one clinical isolate of the microorganism;
correlating the third data set with the second data set and statistically analyzing the correlation;
determining the genetic variations in the at least one clinical isolate of the microorganism associated with antimicrobial drug, e.g. antibiotic, resistance; and
determining a second pan-genome using the first pan-genome as well as the genomes used for constructing it and the first data set of at least one nucleic acid sequence of the at least one clinical isolate of the microorganism.

Further disclosed is a method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations correlated to antimicrobial resistance and/or susceptibility by comparison to a first pan-genome, wherein the first pan-genome is obtained at least partially using a plurality of clinical isolates of the microorganism for which genetic variations are correlated to antimicrobial resistance and/or susceptibility;
determining the genetic variations in the at least one clinical isolate of the microorganism associated with antimicrobial drug resistance; and
determining a second pan-genome using the first pan-genome as well as the genomes used for constructing it and the first data set of at least one nucleic acid sequence of the at least one clinical isolate of the microorganism.

A further aspect of the present invention relates to a method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations correlated to antimicrobial resistance and/or susceptibility by comparison to a first pan-genome, wherein the first pan-genome is obtained at least partially using a plurality of clinical isolates of the microorganism for which genetic variations are correlated to antimicrobial resistance and/or susceptibility; and
determining the genetic variations in the at least one clinical isolate of the microorganism associated with antimicrobial drug resistance.

Also disclosed is a method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations not correlated to antimicrobial resistance and/or susceptibility by comparison to a first pan-genome to obtain a third data set of genetic variants, and analyzing the at least one nucleic acid sequence of the first data set for genetic variations correlated to anti-microbial resistance and/or susceptibility by comparison to the first pan-genome, wherein the first pan-genome is obtained at least partially using a plurality of clinical isolates of the microorganism for which genetic variations are correlated to antimicrobial resistance and/or susceptibility;
providing a second data set of antimicrobial drug resistance and/or susceptibility of the at least one clinical isolate of the microorganism;
correlating the third data set with the second data set and statistically analyzing the correlation;
determining the genetic variations in the at least one clinical isolate of the microorganism associated with antimicrobial drug resistance; and
determining a second pan-genome using the first pan-genome as well as the genomes used for constructing it and the first data set of at least one nucleic acid sequence of the at least one clinical isolate of the microorganism.

A further aspect of the present invention is directed to a method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations not correlated to antimicrobial resistance and/or susceptibility by comparison to a first pan-genome to obtain a third data set of genetic variants, and analyzing the at least one nucleic acid sequence of the first data set for genetic variations correlated to anti-microbial resistance and/or susceptibility by comparison to the first pan-genome, wherein the first pan-genome is obtained at least partially using a plurality of clinical isolates of the microorganism for which genetic variations are correlated to antimicrobial resistance and/or susceptibility;
providing a second data set of antimicrobial drug resistance and/or susceptibility of the at least one clinical isolate of the microorganism;
correlating the third data set with the second data set and statistically analyzing the correlation; and
determining the genetic variations in the at least one clinical isolate of the microorganism associated with antimicrobial drug resistance.

In addition, a pan-genome is disclosed, wherein the pan-genome is for *Escherichia coli* and is obtained using at least 150 clinical isolates, preferably at least 250 clinical isolates; or wherein the pan-genome is for *Acinetobacter baumanii* and is obtained using at least 150 clinical isolates, preferably at least 180 clinical isolates; or wherein the pan-genome is for *Citrobacter koseri* and is obtained using at least 35 clinical isolates, preferably at least 55 clinical isolates; or wherein the pan-genome is for *Enterobacter aerogenes* and is obtained using at least 120 clinical isolates, preferably at least 160 clinical isolates; or wherein the pan-genome is for *Enterobacter cloacae* and is obtained using at least 100 clinical isolates, preferably at least 150 clinical isolates; or wherein the pan-genome is for *Klebsiella oxytoca* and is obtained using at least 90 clinical isolates, preferably at least 120 clinical isolates; or wherein the pan-genome is for *Klebsiella pneumoniae* and is obtained using at least 160 clinical isolates, preferably at least 250 clinical isolates; or wherein the pan-genome is for *Morganella morganii* and is obtained using at least 70 clinical isolates, preferably at least 120 clinical isolates; or wherein the pan-genome is for *Proteus mirabilis* and is obtained using at least 120 clinical isolates, preferably at least 140 clinical isolates; or wherein the pan-genome is for *Pseudomonas aeruginosa* and is obtained using at least 100 clinical isolates, preferably at least 140 clinical isolates; or wherein the pan-genome is for *Salmonella enterica* and is obtained using at least 130 clinical isolates, preferably at least 190 clinical isolates; or wherein the pan-genome is for *Staphylococcus aureus* and is obtained using at least 180 clinical isolates, preferably at least 260 clinical isolates; or wherein the pan-genome is for *Serratia marcescens* and is obtained using at least 230 clinical isolates, preferably at least 330 clinical isolates; or wherein the pan-genome is for *Shigella boydii* and is obtained using at least 60 clinical isolates, preferably at least 75 clinical isolates; or wherein the pan-genome is for *Shigella flexneri* and is obtained using at least 50 clinical isolates, preferably at least 80 clinical isolates; or wherein the pan-genome is for *Shigella sonnei* and is obtained using at least 60 clinical isolates, preferably at least 80 clinical isolates; or wherein the pan-genome is for *Stenotrophomonas maltophilia* and is obtained using at least 125 clinical isolates, preferably at least 240 clinical isolates.

Disclosed is further the use of a pan-genome of the invention for the determination of bacterial resistance in a sample from a patient.

The invention also relates to a method of determining an infection of a patient with a microorganism, comprising the steps of:
a) obtaining or providing a sample containing or suspected of containing a microorganism from the patient;
b) determining the presence of at least one genetic variation in at least one genetic sequence of the microorganism, as determined by the present methods of determining an anti-microbial, e.g. antibiotic, drug resistance profile, wherein the presence of said at least one genetic variation is indicative of an infection with an antimicrobial drug resistant microorganism in said patient.

Additionally disclosed is a method of selecting a treatment of a patient suffering from an infection with a potentially resistant microorganism, comprising the steps of:
a) obtaining or providing a sample containing or suspected of containing a microorganism from the patient;
b) determining the presence of at least one genetic variation in at least one genetic sequence of the microorganism, as determined by the present methods of determining an anti-microbial, e.g. antibiotic, drug resistance profile, wherein the presence of said at least one genetic variation is indicative of an infection with an antimicrobial drug resistant microorganism in said patient;
c) identifying said at least one or more antimicrobial drugs; and
d) selecting one or more antimicrobial drugs different from the ones identified in step c) and being suitable for the treatment of the infection with the microorganism.

Moreover, the present invention is directed to a computer program product comprising computer executable instructions which, when executed, perform a method of the present invention.

Even further aspects and embodiments of the invention are disclosed in the dependent claims and can be taken from the following description and examples, without being limited thereto.

### Detailed description of the invention

### Definitions

Unless defined otherwise, technical and scientific terms used herein have the same meaning as commonly understood by one of ordinary skill in the art to which this invention belongs.

Susceptibility herein means that isolates are inhibited by a certain concentration of an antimicrobial agent, whereas resistance means that isolates are not inhibited

An "antimicrobial drug" in the present invention refers to a group of drugs that includes antibiotics, antifungals, antiprotozoals, and antivirals. According to certain embodiments, the antimicrobial drug is an antibiotic.

The term "nucleic acid molecule" refers to a macromolecule comprising nucleotides, particularly a polynucleotide molecule having a defined sequence. It comprises DNA molecules, RNA molecules, nucleotide analog molecules and combinations and derivatives thereof, such as DNA molecules or RNA molecules with incorporated nucleotide analogs or cDNA.

The term "nucleic acid sequence information" relates to information which can be derived from the sequence of a nucleic acid molecule, i.e. the nucleic acid sequence, such as the sequence itself or a variation in the sequence as compared to a pan-genome. A genetic sequence can thereby encompass coding as well as non-coding parts. The whole genetic material of a microorganism thereby makes up the genome.

The term "genetic variation", which also can be termed "mutation", relates in certain embodiments to a variation in the sequence as compared to a pan-genome as reference sequence, particularly for which it is essentially known or assured, e.g. known, that the sequence does essentially not or not contain genetic variations, e.g. by comprising isolates obtained from samples with no antimicrobial, e.g. antibiotic, resistance, and/or which is not correlated to antimicrobial resistance and/or susceptibility, e.g. as in the fourth and sixth aspect of the present invention. Such a reference sequence can be e.g. determined from a multitude of predominant wild type organisms or other reference organisms, e.g. a defined and known bacterial strain or substrain, and pan-genomes obtained therefrom. A mutation is for example a deletion of one or multiple nucleotides, an insertion of one or multiple nucleotides, or substitution of one or multiple nucleotides, duplication of one or a sequence of multiple nucleotides, translocation of one or a sequence of multiple nucleotides, e.g. also a single nucleotide poly-morphism (SNP). The term "single nucleotide polymorphism" (SNP) is thereby synonymous to the term "single nucleotide variant" (SNV), and both refer to the same. According to certain embodiments, the term "genetic variation" encompasses SNPs as well as structural variations.

In certain embodiments, a genetic variation relates to a variation in the sequence as compared to a pan-genome as reference sequence, wherein the reference sequence contains known genetic variations which are correlated to antimicrobial, e.g. antibiotic, resistance and/or susceptibility, e.g. as in the fifth aspect. This means that the pan-genome is obtained using isolates for which it is known that they correlate with antimicrobial, e.g. antibiotic, resistance and/or susceptibility.

In the present invention a pan-genome can thus be one wherein the sequences are not correlated to antimicrobial resistance and/or susceptibility, one wherein the sequences are correlated to antimicrobial resistance and/or susceptibility, or one that comprises both sequences that are correlated to antimicrobial resistance and/or susceptibility and sequences that are not correlated to antimicrobial resistance and/or susceptibility.

In the context of the present invention a "sample" is a sample which comprises at least one nucleic acid molecule, particularly from a bacterial microorganism. Examples for samples are: cells, tissue, biopsy specimens, body fluids such as blood, urine, saliva, sputum, plasma, serum, cell culture supernatant, swab sample and others. According to certain embodiments, the sample is a patient sample (clinical isolate).

New and highly efficient methods of sequencing nucleic acids referred to as next generation sequencing have opened the possibility of large scale genomic analysis. The term "next generation sequencing" or "high throughput sequencing" refers to methods achieving a higher throughput in sequencing, e.g. high-throughput sequencing technologies that parallelize the sequencing process, producing thousands or millions of sequences at once, or methods producing longer reads and are read out faster. Examples include Massively Parallel Signature Sequencing (MPSS), Polony sequencing, 454 pyrosequencing, Illumina (Solexa) sequencing, SOLiD sequencing, Ion semiconductor sequencing, DNA nanoball sequencing, Helioscope(TM) single molecule sequencing, Single Molecule SMRT(TM) sequencing, Single Molecule real time (RNAP) sequencing, Nanopore DNA sequencing, Sequencing By Hybridization, Amplicon Sequencing, GnuBio.

Within the present description the term "microorganism" comprises the term microbe. The type of microorganism is not particularly restricted, unless noted otherwise or obvious, and, for example, comprises bacteria, viruses, fungi, microscopic algae und protozoa, as well as combinations thereof. According to certain embodiments, it refers to one or more bacterial species, being either Gram-negative or Gram-positive, e.g. one or more of Acinetobacter, e.g. *Acinetobacter baumannii,* Citrobacter, e.g. *Citrobacter koseri,* Escherichia, e.g. *E.coli,* Enterobacter, e.g. *Enterobacter aerogenes* and/or *Enterobacter cloacae,* Klebsiella, e.g. *Klebsiella oxytoca* and/or *Klebsiella pneumoniae,* Morganella, e.g. *Morganella morganii,* Proteus, e.g. *Proteus mirabilis,* Pseudomonas, e.g. *Pseudomonas aeruginosa,* Salmonella, e.g. *Salmonella enterica,* Serratia, e.g. *Serratia marcescens,* Shigella, e.g. *Shigella boydii, Shigella flexneri* and/or *Shigella sonnei,* Stenotrophomonas, e.g. *Stenotrophomonas maltophilia,* and/or Staphylococcus species, e.g. *Staphylococcus aureus.*

A reference to a microorganism or microorganisms in the present description comprises a reference to one microorganism as well a plurality of microorganisms, e.g. two, three, four, five, six, seven, eight, nine, ten, eleven, twelve, thirteen, fourteen, fifteen, sixteen, seventeen, or more microorganisms.

A vertebrate within the present invention refers to animals having a vertebrae, which includes mammals - including humans, birds, reptiles, amphibians and fishes. The present invention thus is not only suitable for human medicine, but also for veterinary medicine.

According to certain embodiments, the patient in the present methods is a vertebrate, more preferably a mammal and most preferred a human patient.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

Regarding the dosage of the antimicrobial, e.g. antibiotic, drugs, it is referred to the established principles of pharmacology in human and veterinary medicine. For example, Forth, Henschler, Rummel "Allgemeine und spezielle Pharmakologie und Toxikologie", 9th edition, 2005, pp. 781-919 might be used as a guideline. Regarding the formulation of a ready-to-use medicament, reference is made to "Remington, The Science and Practice of Pharmacy", 22nd edition, 2013, pp. 777-1070.

Assembling of a nucleic acid, e.g. gene, sequence can be carried out by any known method and is not particularly limited.

According to certain embodiments, mutations, respectively genetic variations, that were found using alignments can also be compared or matched with alignment-free methods, e.g. for detecting single base exchanges, for example based on contigs that were found by assemblies. For example, reads obtained from sequencing can be assembled to contigs and the contigs can be compared to each other.

In the description, the term "structural variations" is used equivalently to the term "structural changes", and both refer to the same phenomenon within the scope of this invention.

A structural variation comprising a change in the nucleic acid sequence comprising more than one base refers to a structural variation wherein at least two bases, preferably at least four bases, in a nucleic acid sequence of a microorganism that are adjacent are changed, and can refer to e.g. a deletion of multiple (2, e.g. 4, or more) nucleotides, an insertion of multiple (2, e.g. 4, or more) nucleotides, a substitution of multiple (2, e.g. 4, or more) nucleotides, a duplication of a sequence of multiple (2, e.g. 4, or more) nucleotides, or a translocation of a sequence of multiple (2, e.g. 4, or more) nucleotides. According to certain embodiments, a structural variation affects a sequence length of at least about 50 bases, preferably at least about 100 bases, further preferably at least about 1 Kb (= 1000 bases). According to certain embodiments, a structural variation affects a sequence length of at most 300 Mb (Mega base = 1000000 bases), e.g. of at most 30 Mb, e.g. of at most 3Mb. In case the term "structural variation refers to a change in the nucleic acid sequence of 4 or more bases, e.g. at least about 50 bases, preferably at least about 100 bases, further preferably at least about 1 Kb, the term single nucleotide polymorphism can be understood to include also small indels (insertions or deletions) of up to at most 3 bases, e.g. up to two bases. According to certain embodiments, a structural variation can comprise bigger parts sections of the nucleic acid sequence, e.g. at least one whole gene in the nucleic acid sequence of the microorganism, or even more genes in an open reading frame. According to certain embodiments, structural variations refer to inclusion of repetitive elements, copy number variations (gains and losses of single genes or larger parts of chromosomes), gene fusions, translocations and other more rare events. According to certain embodiments, at least one inclusion of repetitive elements, one copy number variation (gains and losses of single genes or larger parts of chromosomes), one gene fusion, and/or translocation of single genes or larger parts of chromosomes is observed in the present methods as a structural variation. Structural variations can e.g. include inclusion of repetitive elements, copy number variations (gains and losses of single genes or larger parts of chromosomes), gene fusions, translocations, inclusion/addition of new genes, and other more rare events.

A single nucleotide polymorphism (SNP) refers within the scope of the invention to a variation in a single nucleotide within a nucleic acid sequence, which can result from e.g. an addition, deletion, substitution, insertion or translocation of a single nucleotide.

In the present invention, a reference sequence is not particularly limited, as long as it is useful as a reference for one or more unknown nucleic acid sequences in one or more samples. It is represented by one or more pan-genomes, i.e. alignment is carried out using a pan-genome. A pan-genome, also referred to as supra-genome, can describe the full complement of genes in a clade, e.g. a certain species in bacteria, which can vary among related strains. According to certain embodiments, the reference sequences comprise one or more centroids, wherein a centroid is a representative of a gene group/family/cluster of a genome, e.g. of a microorganism. Centroids can be for example extracted from the database MetaRef (http://metaref.org/). After the extraction the data from the MetaRef database can be updated continually for further experiments. A list of centroids can be extracted for each organism separately or as a whole. The centroid information, e.g. for annotation, can be extracted from databases like IMG (http://img.jgi.doe.gov/) or NCBI.

In a first aspect, the present invention relates to a data bank comprising at least one pan-genome of one of the following bacterial species: *Escherichia coli, Acinetobacter baumanii, Citrobacter koseri, Enterobacter aerogenes, Enterobacter cloacae, Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas maltophilia,* wherein the pan-genome of *Escherichia coli* comprises or consists of SEQ ID NO 1 - 41296 or comprises or consists of sequences that are at least 90% identical to SEQ ID NO 1 - 41296 or comprises at least 90% of SEQ ID NO 1 - 41296, the pan-genome of *Acinetobacter baumanii* comprises or consists of SEQ ID NO 41297 - 71315 or comprises or consists of sequences that are at least 90% identical to SEQ ID NO 41297 - 71315 or comprises at least 90% of SEQ ID NO 41297 - 71315, the pan-genome of *Citrobacter koseri* comprises or consists of SEQ ID NO 71316 - 86840 or comprises or consists of sequences that are at least 90% identical to SEQ ID NO 71316 - 86840 or comprises at least 90% of SEQ ID NO 71316 - 86840, the pan-genome of *Enterobacter aerogenes* comprises or consists of SEQ ID NO 86841 - 113784 or comprises or consists of sequences that are at least 90% identical to SEQ ID NO 86841 - 113784 or comprises at least 90% of SEQ ID NO 86841 - 113784, the pan-genome of *Enterobacter cloacae* comprises or consists of SEQ ID NO 113785 - 171921 or comprises or consists of sequences that are at least 90% identical to SEQ ID NO 113785 - 171921 or comprises at least 90% of SEQ ID NO 113785 - 171921, the pan-genome of *Klebsiella oxytoca* comprises or consists of SEQ ID NO 171922 - 209528 or comprises or consists of sequences that are at least 90% identical to SEQ ID NO 171922 - 209528 or comprises at least 90% of SEQ ID NO 171922 - 209528, the pan-genome of *Klebsiella pneumoniae* comprises or consists of SEQ ID NO 209529 - 273591 or comprises or consists of sequences that are at least 90% identical to SEQ ID NO 209529 - 273591 or comprises at least 90% of SEQ ID NO 209529 - 273591, the pan-genome of *Morganella morganii* comprises or consists of SEQ ID NO 273592 - 293457 or comprises or consists of sequences that are at least 90% identical to SEQ ID NO 273592 - 293457 or comprises at least 90% of SEQ ID NO 273592 - 293457, the pan-genome of *Proteus mirabilis* comprises or consists of SEQ ID NO 293458 - 318000 or comprises or consists of sequences that are at least 90% identical to SEQ ID NO 293458 - 318000 or comprises at least 90% of SEQ ID NO 293458 - 318000, the pan-genome of *Pseudomonas aeruginosa* comprises or consists of SEQ ID NO 318001 - 368053 or comprises or consists of sequences that are at least 90% identical to SEQ ID NO 318001 - 368053 or comprises at least 90% of SEQ ID NO 318001 - 368053, the pan-genome of *Salmonella enterica* comprises or consists of SEQ ID NO 368054 - 404774 or comprises or consists of sequences that are at least 90% identical to SEQ ID NO 368054 - 404774 or comprises at least 90% of SEQ ID NO 368054 - 404774, the pan-genome of *Staphylococcus aureus* comprises or consists of SEQ ID NO 404775 - 421990 or comprises or consists of sequences that are at least 90% identical to SEQ ID NO 404775 - 421990 or comprises at least 90% of SEQ ID NO 404775 - 421990, the pan-genome of *Serratia marcescens* comprises or consists of SEQ ID NO 421991 - 457642 or comprises or consists of sequences that are at least 90% identical to SEQ ID NO 421991 - 457642 or comprises at least 90% of SEQ ID NO 421991 - 457642, the pan-genome of *Shigella boydii* comprises or consists of SEQ ID NO 457643 - 470165 or comprises or consists of sequences that are at least 90% identical to SEQ ID NO 457643 - 470165 or comprises at least 90% of SEQ ID NO 457643 - 470165, the pan-genome of *Shigella flexneri* comprises or consists of SEQ ID NO 470166 - 481587 or comprises or consists of sequences that are at least 90% identical to SEQ ID NO 470166 - 481587 or comprises at least 90% of SEQ ID NO 470166 - 481587, the pan-genome of *Shigella sonnei* comprises or consists of SEQ ID NO 481588 - 492644 or comprises or consists of sequences that are at least 90% identical to SEQ ID NO 481588 - 492644 or comprises at least 90% of SEQ ID NO 481588 - 492644, and the pan-genome of *Stenotrophomonas maltophilia* comprises or consists of SEQ ID NO 492645 - 537429 or comprises or consists of sequences that are at least 90% identical to SEQ ID NO 492645 - 537429 or comprises at least 90% of SEQ ID NO 492645 - 537429.

According to certain embodiments, the data bank comprises at least one pan-genome of one of the following bacterial species: *Escherichia coli, Acinetobacter baumanii, Citrobacter koseri, Enterobacter aerogenes, Enterobacter cloacae, Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas maltophilia,* wherein the pan-genome of *Escherichia coli* comprises or consists of SEQ ID NO 1 - 41296 or comprises or consists of sequences that are at least 95% identical to SEQ ID NO 1 - 41296 or comprises at least 95% of SEQ ID NO 1 - 41296, the pan-genome of *Acinetobacter baumanii* comprises or consists of SEQ ID NO 41297 - 71315 or comprises or consists of sequences that are at least 95% identical to SEQ ID NO 41297 - 71315 or comprises at least 95% of SEQ ID NO 41297 - 71315, the pan-genome of *Citrobacter koseri* comprises or consists of SEQ ID NO 71316 - 86840 or comprises or consists of sequences that are at least 95% identical to SEQ ID NO 71316 - 86840 or comprises at least 95% of SEQ ID NO 71316 - 86840, the pan-genome of *Enterobacter aerogenes* comprises or consists of SEQ ID NO 86841 - 113784 or comprises or consists of sequences that are at least 95% identical to SEQ ID NO 86841 - 113784 or comprises at least 95% of SEQ ID NO 86841 - 113784, the pan-genome of *Enterobacter cloacae* comprises or consists of SEQ ID NO 113785 - 171921 or comprises or consists of sequences that are at least 95% identical to SEQ ID NO 113785 - 171921 or comprises at least 95% of SEQ ID NO 113785 - 171921, the pan-genome of *Klebsiella oxytoca* comprises or consists of SEQ ID NO 171922 - 209528 or comprises or consists of sequences that are at least 95% identical to SEQ ID NO 171922 - 209528 or comprises at least 95% of SEQ ID NO 171922 - 209528, the pan-genome of *Klebsiella pneumoniae* comprises or consists of SEQ ID NO 209529 - 273591 or comprises or consists of sequences that are at least 95% identical to SEQ ID NO 209529 - 273591 or comprises at least 95% of SEQ ID NO 209529 - 273591, the pan-genome of *Morganella morganii* comprises or consists of SEQ ID NO 273592 - 293457 or comprises or consists of sequences that are at least 95% identical to SEQ ID NO 273592 - 293457 or comprises at least 95% of SEQ ID NO 273592 - 293457, the pan-genome of *Proteus mirabilis* comprises or consists of SEQ ID NO 293458 - 318000 or comprises or consists of sequences that are at least 95% identical to SEQ ID NO 293458 - 318000 or comprises at least 95% of SEQ ID NO 293458 - 318000, the pan-genome of *Pseudomonas aeruginosa* comprises or consists of SEQ ID NO 318001 - 368053 or comprises or consists of sequences that are at least 95% identical to SEQ ID NO 318001 - 368053 or comprises at least 95% of SEQ ID NO 318001 - 368053, the pan-genome of *Salmonella enterica* comprises or consists of SEQ ID NO 368054 - 404774 or comprises or consists of sequences that are at least 95% identical to SEQ ID NO 368054 - 404774 or comprises at least 95% of SEQ ID NO 368054 - 404774, the pan-genome of *Staphylococcus aureus* comprises or consists of SEQ ID NO 404775 - 421990 or comprises or consists of sequences that are at least 95% identical to SEQ ID NO 404775 - 421990 or comprises at least 95% of SEQ ID NO 404775 - 421990, the pan-genome of *Serratia marcescens* comprises or consists of SEQ ID NO 421991 - 457642 or comprises or consists of sequences that are at least 95% identical to SEQ ID NO 421991 - 457642 or comprises at least 95% of SEQ ID NO 421991 - 457642, the pan-genome of *Shigella boydii* comprises or consists of SEQ ID NO 457643 - 470165 or comprises or consists of sequences that are at least 95% identical to SEQ ID NO 457643 - 470165 or comprises at least 95% of SEQ ID NO 457643 - 470165, the pan-genome of *Shigella flexneri* comprises or consists of SEQ ID NO 470166 - 481587 or comprises or consists of sequences that are at least 95% identical to SEQ ID NO 470166 - 481587 or comprises at least 95% of SEQ ID NO 470166 - 481587, the pan-genome of *Shigella sonnei* comprises or consists of SEQ ID NO 481588 - 492644 or comprises or consists of sequences that are at least 95% identical to SEQ ID NO 481588 - 492644 or comprises at least 95% of SEQ ID NO 481588 - 492644, and the pan-genome of *Stenotrophomonas maltophilia* comprises or consists of SEQ ID NO 492645 - 537429 or comprises or consists of sequences that are at least 95% identical to SEQ ID NO 492645 - 537429 or comprises at least 95% of SEQ ID NO 492645 - 537429.

According to certain embodiments, the data bank comprises at least one pan-genome of one of the following bacterial species: *Escherichia coli, Acinetobacter baumanii, Citrobacter koseri, Enterobacter aerogenes, Enterobacter cloacae, Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas maltophilia,*
wherein the pan-genome of *Escherichia coli* comprises or consists of SEQ ID NO 1 - 41296 or comprises or consists of sequences that are at least 99% identical to SEQ ID NO 1 - 41296 or comprises at least 99% of SEQ ID NO 1 - 41296, the pan-genome of *Acinetobacter baumanii* comprises or consists of SEQ ID NO 41297 - 71315 or comprises or consists of sequences that are at least 99% identical to SEQ ID NO 41297 - 71315 or comprises at least 99% of SEQ ID NO 41297 - 71315, the pan-genome of *Citrobacter koseri* comprises or consists of SEQ ID NO 71316 - 86840 or comprises or consists of sequences that are at least 99% identical to SEQ ID NO 71316 - 86840 or comprises at least 99% of SEQ ID NO 71316 - 86840, the pan-genome of *Enterobacter aerogenes* comprises or consists of SEQ ID NO 86841 - 113784 or comprises or consists of sequences that are at least 99% identical to SEQ ID NO 86841 - 113784 or comprises at least 99% of SEQ ID NO 86841 - 113784, the pan-genome of *Enterobacter cloacae* comprises or consists of SEQ ID NO 113785 - 171921 or comprises or consists of sequences that are at least 99% identical to SEQ ID NO 113785 - 171921 or comprises at least 99% of SEQ ID NO 113785 - 171921, the pan-genome of *Klebsiella oxytoca* comprises or consists of SEQ ID NO 171922 - 209528 or comprises or consists of sequences that are at least 99% identical to SEQ ID NO 171922 - 209528 or comprises at least 99% of SEQ ID NO 171922 - 209528, the pan-genome of *Klebsiella pneumoniae* comprises or consists of SEQ ID NO 209529 - 273591 or comprises or consists of sequences that are at least 99% identical to SEQ ID NO 209529 - 273591 or comprises at least 99% of SEQ ID NO 209529 - 273591, the pan-genome of *Morganella morganii* comprises or consists of SEQ ID NO 273592 - 293457 or comprises or consists of sequences that are at least 99% identical to SEQ ID NO 273592 - 293457 or comprises at least 99% of SEQ ID NO 273592 - 293457, the pan-genome of *Proteus mirabilis* comprises or consists of SEQ ID NO 293458 - 318000 or comprises or consists of sequences that are at least 99% identical to SEQ ID NO 293458 - 318000 or comprises at least 99% of SEQ ID NO 293458 - 318000, the pan-genome of *Pseudomonas aeruginosa* comprises or consists of SEQ ID NO 318001 - 368053 or comprises or consists of sequences that are at least 99% identical to SEQ ID NO 318001 - 368053 or comprises at least 99% of SEQ ID NO 318001 - 368053, the pan-genome of *Salmonella enterica* comprises or consists of SEQ ID NO 368054 - 404774 or comprises or consists of sequences that are at least 99% identical to SEQ ID NO 368054 - 404774 or comprises at least 99% of SEQ ID NO 368054 - 404774, the pan-genome of *Staphylococcus aureus* comprises or consists of SEQ ID NO 404775 - 421990 or comprises or consists of sequences that are at least 99% identical to SEQ ID NO 404775 - 421990 or comprises at least 99% of SEQ ID NO 404775 - 421990, the pan-genome of *Serratia marcescens* comprises or consists of SEQ ID NO 421991 - 457642 or comprises or consists of sequences that are at least 99% identical to SEQ ID NO 421991 - 457642 or comprises at least 99% of SEQ ID NO 421991 - 457642, the pan-genome of *Shigella boydii* comprises or consists of SEQ ID NO 457643 - 470165 or comprises or consists of sequences that are at least 99% identical to SEQ ID NO 457643 - 470165 or comprises at least 99% of SEQ ID NO 457643 - 470165, the pan-genome of *Shigella flexneri* comprises or consists of SEQ ID NO 470166 - 481587 or comprises or consists of sequences that are at least 99% identical to SEQ ID NO 470166 - 481587 or comprises at least 99% of SEQ ID NO 470166 - 481587, the pan-genome of *Shigella sonnei* comprises or consists of SEQ ID NO 481588 - 492644 or comprises or consists of sequences that are at least 99% identical to SEQ ID NO 481588 - 492644 or comprises at least 99% of SEQ ID NO 481588 - 492644, and the pan-genome of *Stenotrophomonas maltophilia* comprises or consists of SEQ ID NO 492645 - 537429 or comprises or consists of sequences that are at least 99% identical to SEQ ID NO 492645 - 537429 or comprises at least 99% of SEQ ID NO 492645 - 537429.

According to certain embodiments, the data bank comprises at least one pan-genome of one of the following bacterial species: *Escherichia coli, Acinetobacter baumanii, Citrobacter koseri, Enterobacter aerogenes, Enterobacter cloacae, Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas maltophilia,*
wherein the pan-genome of *Escherichia coli* comprises or consists of SEQ ID NO 1 - 41296, the pan-genome of *Acinetobacter baumanii* comprises or consists of SEQ ID NO 41297 - 71315, the pan-genome of *Citrobacter koseri* comprises or consists of SEQ ID NO 71316 - 86840, the pan-genome of *Enterobacter aerogenes* comprises or consists of SEQ ID NO 86841 - 113784, the pan-genome of *Enterobacter cloacae* comprises or consists of SEQ ID NO 113785 - 171921, the pan-genome of *Klebsiella oxytoca* comprises or consists of SEQ ID NO 171922 - 209528, the pan-genome of *Klebsiella pneumoniae* comprises or consists of SEQ ID NO 209529 - 273591, the pan-genome of *Morganella morganii* comprises or consists of SEQ ID NO 273592 - 293457, the pan-genome of *Proteus mirabilis* comprises or consists of SEQ ID NO 293458 - 318000, the pan-genome of *Pseudomonas aeruginosa* comprises or consists of SEQ ID NO 318001 - 368053, the pan-genome of *Salmonella enterica* comprises or consists of SEQ ID NO 368054 - 404774, the pan-genome of *Staphylococcus aureus* comprises or consists of SEQ ID NO 404775 - 421990, the pan-genome of *Serratia marcescens* comprises or consists of SEQ ID NO 421991 - 457642, the pan-genome of *Shigella boydii* comprises or consists of SEQ ID NO 457643 - 470165, the pan-genome of *Shigella flexneri* comprises or consists of SEQ ID NO 470166 - 481587, the pan-genome of *Shigella sonnei* comprises or consists of SEQ ID NO 481588 - 492644, and the pan-genome of *Stenotrophomonas maltophilia* comprises or consists of SEQ ID NO 492645 - 537429.

According to certain embodiments, the data bank comprises 2, 3, 4, 5, 6, 7, 8, 9, 10, 11, 12, 13, 14, 15, 16, or 17 of the pan-genomes.

The pan-genomes comprising the above SEQ ID NOs (sequence accession identifier numbers) have been found to be stable with regard to their core genome and with regard to the addition of new genes.

In order to show the "convergence" of the pan-genomes, they were iteratively plotted with the addition of each new genome of a new sample to the existing pan-genome, and analyzed in each turn for the addition of "new" genes as well as for a stability of the "core" genes (or conserved genes), i.e. genes present in 90% of all samples. The number of "new" genes decreases significantly with an increasing number of genomes and approached a value near 0 fast, whereas the size of the "core" genome (gene present in 90% of samples) only stabilized after a certain number of genomes was added. In the plot of the core genome, initially regular fluctuations were observed due to the continued addition of new genes as well as the loss of other genes in the core genome, which were dampened until the regular fluctuations ceased to exist, at which point a stability of the core genome is assumed to exist. The stability of the core genome was reached for different bacterial species with different numbers of added genomes. For the pan-genomes, first a stop of the regularity of the fluctuations was observed, and then at a second number of genomes of clinical isolates used for obtaining the pan-genome fluctuations essentially were minimized so that a stable core genome was observed. The respective numbers observed for the different bacterial species were as follows:
*Escherichia coli:* at least 150 clinical isolates, preferably at least 250 clinical isolates;
*Acinetobacter baumanii:* at least 150 clinical isolates, preferably at least 180 clinical isolates;
*Citrobacter koseri:* at least 35 clinical isolates, preferably at least 55 clinical isolates;
*Enterobacter aerogenes:* at least 120 clinical isolates, preferably at least 160 clinical isolates;
*Enterobacter cloacae:* at least 100 clinical isolates, preferably at least 150 clinical isolates;
*Klebsiella oxytoca:* at least 90 clinical isolates, preferably at least 120 clinical isolates;
*Klebsiella pneumoniae:* at least 160 clinical isolates, preferably at least 250 clinical isolates;
*Morganella morganii:* at least 70 clinical isolates, preferably at least 120 clinical isolates;
*Proteus mirabilis:* at least 120 clinical isolates, preferably at least 140 clinical isolates;
*Pseudomonas aeruginosa:* at least 100 clinical isolates, preferably at least 140 clinical isolates;
*Salmonella enterica:* at least 130 clinical isolates, preferably at least 190 clinical isolates;
*Staphylococcus aureus:* at least 180 clinical isolates, preferably at least 260 clinical isolates;
*Serratia marcescens:* at least 230 clinical isolates, preferably at least 330 clinical isolates;
*Shigella boydii:* at least 60 clinical isolates, preferably at least 75 clinical isolates;
*Shigella flexneri:* at least 50 clinical isolates, preferably at least 80 clinical isolates;
*Shigella sonnei:* at least 60 clinical isolates, preferably at least 80 clinical isolates;
*Stenotrophomonas maltophilia:* at least 125 clinical isolates, preferably at least 240 clinical isolates.

According to certain embodiments, the data bank can be at a remote location and can be queried from a local client.

The present data banks, e.g. of the first and sixteenth aspect, as well as the present pan genomes, e.g. of the third and seventh aspect, can be used in a variety of applications.

For example, they can be used for the determination of bacterial resistance in a sample from a patient, as will be described in more detail in the following.

Further, they can be also used in the search for a minimal bacterial genome. For examples, 20 conserved genes could be found in all the pan-genomes of the third aspect. Thus, the data banks and pan-genomes can be used to enhance the understanding of the genetic building blocks of different bacteria, as well as for comparison amongst them. In a further step, even minimal, median und maximal building blocks (e.g. a minimal, median and maximum number, sequence, etc.) for each species of bacteria as well as bacteria as a whole can then be defined.

Using the pan-genomes, also variability with regard to single genes and/or the genome as a whole can be shown.

Furthermore, the present data banks and pan-genomes can be used in drug target finding approaches.

Thus, accordingly, respective methods for assisting in finding a minimal bacterial genome, or for finding a minimal bacterial genome, for determining variability with regard to single genes and/or the genome as a whole, for finding new drug targets, etc., using the present data banks and pan-genomes can be formulated. These methods can also be related to computer program products to carry them out, wherein the computer program products comprise the present pan genomes, e.g. of the third and seventh aspect.

In a second aspect the present invention relates to the use of the data bank for the determination of bacterial resistance in a sample from a patient. As the pan-genomes reflect a stable core-genome and a wide variety of genes for each bacterial species, the use of the data bank leads to improved results.

A third aspect of the invention relates to a pan-genome of one of the following bacterial species: *Escherichia coli, Acinetobacter baumanii, Citrobacter koseri, Enterobacter aerogenes, Enterobacter cloacae, Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas maltophilia,* wherein the pan-genome of *Escherichia coli* comprises or consists of SEQ ID NO 1 - 41296 or comprises or consists of sequences that are at least 90% identical to SEQ ID NO 1 - 41296 or comprises at least 90% of SEQ ID NO 1 - 41296, the pan-genome of *Acinetobacter baumanii* comprises or consists of SEQ ID NO 41297 - 71315 or comprises or consists of sequences that are at least 90% identical to SEQ ID NO 41297 - 71315 or comprises at least 90% of SEQ ID NO 41297 - 71315, the pan-genome of *Citrobacter koseri* comprises or consists of SEQ ID NO 71316 - 86840 or comprises or consists of sequences that are at least 90% identical to SEQ ID NO 71316 - 86840 or comprises at least 90% of SEQ ID NO 71316 - 86840, the pan-genome of *Enterobacter aerogenes* comprises or consists of SEQ ID NO 86841 - 113784 or comprises or consists of sequences that are at least 90% identical to SEQ ID NO 86841 - 113784 or comprises at least 90% of SEQ ID NO 86841 - 113784, the pan-genome of *Enterobacter cloacae* comprises or consists of SEQ ID NO 113785 - 171921 or comprises or consists of sequences that are at least 90% identical to SEQ ID NO 113785 - 171921 or comprises at least 90% of SEQ ID NO 113785 - 171921, the pan-genome of *Klebsiella oxytoca* comprises or consists of SEQ ID NO 171922 - 209528 or comprises or consists of sequences that are at least 90% identical to SEQ ID NO 171922 - 209528 or comprises at least 90% of SEQ ID NO 171922 - 209528, the pan-genome of *Klebsiella pneumoniae* comprises or consists of SEQ ID NO 209529 - 273591 or comprises or consists of sequences that are at least 90% identical to SEQ ID NO 209529 - 273591 or comprises at least 90% of SEQ ID NO 209529 - 273591, the pan-genome of *Morganella morganii* comprises or consists of SEQ ID NO 273592 - 293457 or comprises or consists of sequences that are at least 90% identical to SEQ ID NO 273592 - 293457 or comprises at least 90% of SEQ ID NO 273592 - 293457, the pan-genome of *Proteus mirabilis* comprises or consists of SEQ ID NO 293458 - 318000 or comprises or consists of sequences that are at least 90% identical to SEQ ID NO 293458 - 318000 or comprises at least 90% of SEQ ID NO 293458 - 318000, the pan-genome of *Pseudomonas aeruginosa* comprises or consists of SEQ ID NO 318001 - 368053 or comprises or consists of sequences that are at least 90% identical to SEQ ID NO 318001 - 368053 or comprises at least 90% of SEQ ID NO 318001 - 368053, the pan-genome of *Salmonella enterica* comprises or consists of SEQ ID NO 368054 - 404774 or comprises or consists of sequences that are at least 90% identical to SEQ ID NO 368054 - 404774 or comprises at least 90% of SEQ ID NO 368054 - 404774, the pan-genome of *Staphylococcus aureus* comprises or consists of SEQ ID NO 404775 - 421990 or comprises or consists of sequences that are at least 90% identical to SEQ ID NO 404775 - 421990 or comprises at least 90% of SEQ ID NO 404775 - 421990, the pan-genome of *Serratia marcescens* comprises or consists of SEQ ID NO 421991 - 457642 or comprises or consists of sequences that are at least 90% identical to SEQ ID NO 421991 - 457642 or comprises at least 90% of SEQ ID NO 421991 - 457642, the pan-genome of *Shigella boydii* comprises or consists of SEQ ID NO 457643 - 470165 or comprises or consists of sequences that are at least 90% identical to SEQ ID NO 457643 - 470165 or comprises at least 90% of SEQ ID NO 457643 - 470165, the pan-genome of *Shigella flexneri* comprises or consists of SEQ ID NO 470166 - 481587 or comprises or consists of sequences that are at least 90% identical to SEQ ID NO 470166 - 481587 or comprises at least 90% of SEQ ID NO 470166 - 481587, the pan-genome of *Shigella sonnei* comprises or consists of SEQ ID NO 481588 - 492644 or comprises or consists of sequences that are at least 90% identical to SEQ ID NO 481588 - 492644 or comprises at least 90% of SEQ ID NO 481588 - 492644, and the pan-genome of *Stenotrophomonas maltophilia* comprises or consists of SEQ ID NO 492645 - 537429 or comprises or consists of sequences that are at least 90% identical to SEQ ID NO 492645 - 537429 or comprises at least 90% of SEQ ID NO 492645 - 537429.

According to certain embodiments, the invention is directed to a pan-genome of one of the following bacterial species:
*Escherichia coli, Acinetobacter baumanii, Citrobacter koseri, Enterobacter aerogenes, Enterobacter cloacae, Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas maltophilia,*
wherein the pan-genome of *Escherichia coli* comprises or consists of SEQ ID NO 1 - 41296 or comprises or consists of sequences that are at least 95% identical to SEQ ID NO 1 - 41296 or comprises at least 95% of SEQ ID NO 1 - 41296, the pan-genome of *Acinetobacter baumanii* comprises or consists of SEQ ID NO 41297 - 71315 or comprises or consists of sequences that are at least 95% identical to SEQ ID NO 41297 - 71315 or comprises at least 95% of SEQ ID NO 41297 - 71315, the pan-genome of *Citrobacter koseri* comprises or consists of SEQ ID NO 71316 - 86840 or comprises or consists of sequences that are at least 95% identical to SEQ ID NO 71316 - 86840 or comprises at least 95% of SEQ ID NO 71316 - 86840, the pan-genome of *Enterobacter aerogenes* comprises or consists of SEQ ID NO 86841 - 113784 or comprises or consists of sequences that are at least 95% identical to SEQ ID NO 86841 - 113784 or comprises at least 95% of SEQ ID NO 86841 - 113784, the pan-genome of *Enterobacter cloacae* comprises or consists of SEQ ID NO 113785 - 171921 or comprises or consists of sequences that are at least 95% identical to SEQ ID NO 113785 - 171921 or comprises at least 95% of SEQ ID NO 113785 - 171921, the pan-genome of *Klebsiella oxytoca* comprises or consists of SEQ ID NO 171922 - 209528 or comprises or consists of sequences that are at least 95% identical to SEQ ID NO 171922 - 209528 or comprises at least 95% of SEQ ID NO 171922 - 209528, the pan-genome of *Klebsiella pneumoniae* comprises or consists of SEQ ID NO 209529 - 273591 or comprises or consists of sequences that are at least 95% identical to SEQ ID NO 209529 - 273591 or comprises at least 95% of SEQ ID NO 209529 - 273591, the pan-genome of *Morganella morganii* comprises or consists of SEQ ID NO 273592 - 293457 or comprises or consists of sequences that are at least 95% identical to SEQ ID NO 273592 - 293457 or comprises at least 95% of SEQ ID NO 273592 - 293457, the pan-genome of *Proteus mirabilis* comprises or consists of SEQ ID NO 293458 - 318000 or comprises or consists of sequences that are at least 95% identical to SEQ ID NO 293458 - 318000 or comprises at least 95% of SEQ ID NO 293458 - 318000, the pan-genome of *Pseudomonas aeruginosa* comprises or consists of SEQ ID NO 318001 - 368053 or comprises or consists of sequences that are at least 95% identical to SEQ ID NO 318001 - 368053 or comprises at least 95% of SEQ ID NO 318001 - 368053, the pan-genome of *Salmonella enterica* comprises or consists of SEQ ID NO 368054 - 404774 or comprises or consists of sequences that are at least 95% identical to SEQ ID NO 368054 - 404774 or comprises at least 95% of SEQ ID NO 368054 - 404774, the pan-genome of *Staphylococcus aureus* comprises or consists of SEQ ID NO 404775 - 421990 or comprises or consists of sequences that are at least 95% identical to SEQ ID NO 404775 - 421990 or comprises at least 95% of SEQ ID NO 404775 - 421990, the pan-genome of *Serratia marcescens* comprises or consists of SEQ ID NO 421991 - 457642 or comprises or consists of sequences that are at least 95% identical to SEQ ID NO 421991 - 457642 or comprises at least 95% of SEQ ID NO 421991 - 457642, the pan-genome of *Shigella boydii* comprises or consists of SEQ ID NO 457643 - 470165 or comprises or consists of sequences that are at least 95% identical to SEQ ID NO 457643 - 470165 or comprises at least 95% of SEQ ID NO 457643 - 470165, the pan-genome of *Shigella flexneri* comprises or consists of SEQ ID NO 470166 - 481587 or comprises or consists of sequences that are at least 95% identical to SEQ ID NO 470166 - 481587 or comprises at least 95% of SEQ ID NO 470166 - 481587, the pan-genome of *Shigella sonnei* comprises or consists of SEQ ID NO 481588 - 492644 or comprises or consists of sequences that are at least 95% identical to SEQ ID NO 481588 - 492644 or comprises at least 95% of SEQ ID NO 481588 - 492644, and the pan-genome of *Stenotrophomonas maltophilia* comprises or consists of SEQ ID NO 492645 - 537429 or comprises or consists of sequences that are at least 95% identical to SEQ ID NO 492645 - 537429 or comprises at least 95% of SEQ ID NO 492645 - 537429.

According to certain embodiments, the invention is directed to a pan-genome of one of the following bacterial species:
*Escherichia coli, Acinetobacter baumanii, Citrobacter koseri, Enterobacter aerogenes, Enterobacter cloacae, Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas maltophilia,*
wherein the pan-genome of *Escherichia coli* comprises or consists of SEQ ID NO 1 - 41296 or comprises or consists of sequences that are at least 99% identical to SEQ ID NO 1 - 41296 or comprises at least 99% of SEQ ID NO 1 - 41296, the pan-genome of *Acinetobacter baumanii* comprises or consists of SEQ ID NO 41297 - 71315 or comprises or consists of sequences that are at least 99% identical to SEQ ID NO 41297 - 71315 or comprises at least 99% of SEQ ID NO 41297 - 71315, the pan-genome of *Citrobacter koseri* comprises or consists of SEQ ID NO 71316 - 86840 or comprises or consists of sequences that are at least 99% identical to SEQ ID NO 71316 - 86840 or comprises at least 99% of SEQ ID NO 71316 - 86840, the pan-genome of *Enterobacter aerogenes* comprises or consists of SEQ ID NO 86841 - 113784 or comprises or consists of sequences that are at least 99% identical to SEQ ID NO 86841 - 113784 or comprises at least 99% of SEQ ID NO 86841 - 113784, the pan-genome of *Enterobacter cloacae* comprises or consists of SEQ ID NO 113785 - 171921 or comprises or consists of sequences that are at least 99% identical to SEQ ID NO 113785 - 171921 or comprises at least 99% of SEQ ID NO 113785 - 171921, the pan-genome of *Klebsiella oxytoca* comprises or consists of SEQ ID NO 171922 - 209528 or comprises or consists of sequences that are at least 99% identical to SEQ ID NO 171922 - 209528 or comprises at least 99% of SEQ ID NO 171922 - 209528, the pan-genome of *Klebsiella pneumoniae* comprises or consists of SEQ ID NO 209529 - 273591 or comprises or consists of sequences that are at least 99% identical to SEQ ID NO 209529 - 273591 or comprises at least 99% of SEQ ID NO 209529 - 273591, the pan-genome of *Morganella morganii* comprises or consists of SEQ ID NO 273592 - 293457 or comprises or consists of sequences that are at least 99% identical to SEQ ID NO 273592 - 293457 or comprises at least 99% of SEQ ID NO 273592 - 293457, the pan-genome of *Proteus mirabilis* comprises or consists of SEQ ID NO 293458 - 318000 or comprises or consists of sequences that are at least 99% identical to SEQ ID NO 293458 - 318000 or comprises at least 99% of SEQ ID NO 293458 - 318000, the pan-genome of *Pseudomonas aeruginosa* comprises or consists of SEQ ID NO 318001 - 368053 or comprises or consists of sequences that are at least 99% identical to SEQ ID NO 318001 - 368053 or comprises at least 99% of SEQ ID NO 318001 - 368053, the pan-genome of *Salmonella enterica* comprises or consists of SEQ ID NO 368054 - 404774 or comprises or consists of sequences that are at least 99% identical to SEQ ID NO 368054 - 404774 or comprises at least 99% of SEQ ID NO 368054 - 404774, the pan-genome of *Staphylococcus aureus* comprises or consists of SEQ ID NO 404775 - 421990 or comprises or consists of sequences that are at least 99% identical to SEQ ID NO 404775 - 421990 or comprises at least 99% of SEQ ID NO 404775 - 421990, the pan-genome of *Serratia marcescens* comprises or consists of SEQ ID NO 421991 - 457642 or comprises or consists of sequences that are at least 99% identical to SEQ ID NO 421991 - 457642 or comprises at least 99% of SEQ ID NO 421991 - 457642, the pan-genome of *Shigella boydii* comprises or consists of SEQ ID NO 457643 - 470165 or comprises or consists of sequences that are at least 99% identical to SEQ ID NO 457643 - 470165 or comprises at least 99% of SEQ ID NO 457643 - 470165, the pan-genome of *Shigella flexneri* comprises or consists of SEQ ID NO 470166 - 481587 or comprises or consists of sequences that are at least 99% identical to SEQ ID NO 470166 - 481587 or comprises at least 99% of SEQ ID NO 470166 - 481587, the pan-genome of *Shigella sonnei* comprises or consists of SEQ ID NO 481588 - 492644 or comprises or consists of sequences that are at least 99% identical to SEQ ID NO 481588 - 492644 or comprises at least 99% of SEQ ID NO 481588 - 492644, and the pan-genome of *Stenotrophomonas maltophilia* comprises or consists of SEQ ID NO 492645 - 537429 or comprises or consists of sequences that are at least 99% identical to SEQ ID NO 492645 - 537429 or comprises at least 99% of SEQ ID NO 492645 - 537429.

According to certain embodiments, the invention is directed to a pan-genome of one of the following bacterial species:
*Escherichia coli, Acinetobacter baumanii, Citrobacter koseri, Enterobacter aerogenes, Enterobacter cloacae, Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas maltophilia,*
wherein the pan-genome of *Escherichia coli* comprises or consists of SEQ ID NO 1 - 41296, the pan-genome of *Acinetobacter baumanii* comprises or consists of SEQ ID NO 41297 - 71315, the pan-genome of *Citrobacter koseri* comprises or consists of SEQ ID NO 71316 - 86840, the pan-genome of *Enterobacter aerogenes* comprises or consists of SEQ ID NO 86841 - 113784, the pan-genome of *Enterobacter cloacae* comprises or consists of SEQ ID NO 113785 - 171921, the pan-genome of *Klebsiella oxytoca* comprises or consists of SEQ ID NO 171922 - 209528, the pan-genome of *Klebsiella pneumoniae* comprises or consists of SEQ ID NO 209529 - 273591, the pan-genome of *Morganella morganii* comprises or consists of SEQ ID NO 273592 - 293457, the pan-genome of *Proteus mirabilis* comprises or consists of SEQ ID NO 293458 - 318000, the pan-genome of *Pseudomonas aeruginosa* comprises or consists of SEQ ID NO 318001 - 368053, the pan-genome of *Salmonella enterica* comprises or consists of SEQ ID NO 368054 - 404774, the pan-genome of *Staphylococcus aureus* comprises or consists of SEQ ID NO 404775 - 421990, the pan-genome of *Serratia marcescens* comprises or consists of SEQ ID NO 421991 - 457642, the pan-genome of *Shigella boydii* comprises or consists of SEQ ID NO 457643 - 470165, the pan-genome of *Shigella flexneri* comprises or consists of SEQ ID NO 470166 - 481587, the pan-genome of *Shigella sonnei* comprises or consists of SEQ ID NO 481588 - 492644, and the pan-genome of *Stenotrophomonas maltophilia* comprises or consists of SEQ ID NO 492645 - 537429.

In a fourth aspect, the present invention relates to a method of determining an antimicrobial, e.g. antibiotic, drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations by comparison to a first pan-genome to obtain a third data set of genetic variants;
providing a second data set of antimicrobial drug, e.g. antibiotic, resistance and/or susceptibility of the at least one clinical isolate of the microorganism;
correlating the third data set with the second data set and statistically analyzing the correlation; determining the genetic variations in the at least one clinical isolate of the microorganism associated with antimicrobial drug, e.g. antibiotic, resistance; and
determining a second pan-genome using the first pan-genome as well as the genomes used for constructing it and the first data set of at least one nucleic acid sequence of the at least one clinical isolate of the microorganism.

When the second pan-genome is determined, the genomes used for making the first pan-genome are used in addition to the first data set of at least one nucleic acid sequence of the at least one clinical isolate of the microorganism, i.e. the at least one further first genome. This way the number of basis genomes for the pan-genome increases while the basic information from the first pan-genome gets carried over, including the information that e.g. might have led to a removal of a gene from the core genome, the construction of a certain centroid, etc. Also, the criteria set for the first pan-genome, like a certain threshold for constructing it, e.g. regarding protein identity of the product of a certain gene, which can be e.g. set to 90% (as in the present case), will then be carried over to the second pan-genome. This way the pan-genome is self-learning with each additional isolate analyzed. This concept also is reflected in the fifth and sixth aspect, as well as related aspects.

According to certain embodiments, the second pan-genome can be used for analyzing the at least one nucleic acid sequence of the first data set for genetic variations by comparison to the second pan-genome to obtain a third data set of genetic variants, which then can again be correlated, thus improving the determination of the genetic variations. Particularly, genetic variations can be identified more accurately since also genes which are unique to the first data set are then part of the second pan genome, which reduces the noise during the variant calling.

According to certain embodiments, a plurality of at least one nucleic acid sequence of at least one clinical isolate of the microorganism can be used iteratively for constructing subsequent pan-genomes, e.g. a second, third, fourth, etc. pan-genome. In this constructing, it is also possible that some of the plurality of at least one nucleic acid sequence of at least one clinical isolate of the microorganism are not taken into account for constructing a new pan-genome, e.g. when the pan-genome is based on a multitude of clinical isolates that are correlated to antimicrobial resistance and/or susceptibility.

According to certain embodiments, the first pan-genome in this aspect is essentially not correlated or not correlated to antimicrobial resistance and/or susceptibility.

According to certain embodiments, the first pan-genome is a pan-genome of the third aspect.

A simple read out concept for a diagnostic test as described in this aspect can be as follows.

A sample, e.g. blood, from a patient, is used for molecular testing, e.g. using next generation sequencing (NGS), and then a molecular fingerprint is taken, e.g. in case of NGS a sequence of selected genomic/plasmid regions or the whole nucleic acid sequences, e.g. genome, is assembled. This is then compared to a first pan-genome, i.e. selected sequences or the whole sequence are/is compared to the first pan-genome, and genetic variations (SNPs, structural variations like sequence / gene additions/deletions, etc.) in the genome of the at least one clinical isolate, e.g. comprising chromosomal nucleic acid sequences and plasmid nucleic acid sequences, can be correlated with susceptibility/resistance using the first pan-genome. Then the result is reported, which can comprise ID (pathogen identification), i.e. a list of all (pathogenic) species identified in the sample, and AST (antimicrobial susceptibility testing), i.e. a list including a susceptibility /resistance profile for all species listed, based on genetic variations.

According to certain embodiments, statistical analysis in the present method is carried out using Fisher's test with p < 10⁻³, preferably p < 10⁻⁶, preferably p < 10⁻⁹. Also, according to certain embodiments, the method further comprises correlating different genetic sites to each other.

The different steps herein can be suitably carried out, e.g. as described with regard to other methods of the present invention.

According to certain embodiments, obtaining or providing a first data set of at least one nucleic acid sequence of the at least one clinical isolate of the microorganism, preferably a bacterial microorganism, e.g. one or more of Acinetobacter, Citrobacter, Escherichia, Enterobacter, Klebsiella, Morganella, Proteus, Pseudomonas, Salmonella, Serratia, Shigella, Stenotrophomonas and/or Staphylococcus species, e.g. *Escherichia coli, Acinetobacter baumanii, Citrobacter koseri, Enterobacter aerogenes, Enterobacter clo*acae, *Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas* maltophilia, from the patient in the methods of the invention can comprise the following:
A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid sequences can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of the microorganism. For example, sequencing can be carried out using polymerase chain reaction (PCR), particularly multiplex PCR, or high throughput sequencing or next generation sequencing, preferably using high-throughput sequencing. For sequencing, preferably an *in vitro* sample is used.

The data obtained by the sequencing can be in any format, and can then be analyzed as described with regard to the methods of the present invention, particularly in the fourth, fifth, sixth, ninth, tenth, eleventh, fourteenth and/or fifteenth aspect.

A fifth aspect of the present invention relates to a method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations correlated to antimicrobial resistance and/or susceptibility by comparison to a first pan-genome, wherein the first pan-genome is obtained at least partially using a plurality of clinical isolates of the microorganism for which genetic variations are correlated to antimicrobial resistance and/or susceptibility;
determining the genetic variations in the at least one clinical isolate of the microorganism associated with antimicrobial drug resistance; and
determining a second pan-genome using the first pan-genome as well as the genomes used for constructing it and the first data set of at least one nucleic acid sequence of the at least one clinical isolate of the microorganism.

Again, according to certain embodiments, the second pan-genome can be used for analyzing the at least one nucleic acid sequence of the first data set for genetic variations by comparison to the second pan-genome to obtain a third data set of genetic variants, which then can again be correlated, as e.g. in the fourth aspect, thus improving the determination of the genetic variations. Particularly, genetic variations can be identified more accurately since also genes which are unique to the first data set are then part of the second pan genome, which reduces the noise during the variant calling.

In the fifth aspect, the first pan genome is preferably constructed predominantly using a plurality of clinical isolates of the microorganism for which genetic variations are correlated to antimicrobial resistance and/or susceptibility, particularly preferably using only a plurality of clinical isolates of the microorganism for which genetic variations are correlated to antimicrobial resistance and/or susceptibility. This way the first pan-genome is correlated with antimicrobial resistance and/or susceptibility.

According to certain embodiments, a plurality of at least one nucleic acid sequence of at least one clinical isolate of the microorganism can be used iteratively for constructing subsequent pan-genomes, e.g. a second, third, fourth, etc. pan-genome. In this constructing, it is also possible that some of the plurality of at least one nucleic acid sequence of at least one clinical isolate of the microorganism are not taken into account for constructing a new pan-genome, e.g. when the resulting pan-genome would be based on a multitude of clinical isolates that are not correlated to antimicrobial resistance and/or susceptibility.

Other steps can be similar or identical as described in e.g. the fourth aspect or other related methods.

A further, fourteenth, aspect of the present invention relates to a method of determining an antimicrobial drug resistance profile for at least one clinical isolate of a microorganism and for improving a first pan-genome, comprising: obtaining or providing a first data set of at least one nucleic acid sequence of the at least one clinical isolate of the microorganism, wherein optionally at least a part of the nucleic acid sequence of the first data set is assembled; analyzing the at least one nucleic acid sequence of the first data set for genetic variations correlated to antimicrobial resistance and/or susceptibility by comparison to a first pan-genome, wherein the first pan-genome is obtained at least partially using a plurality of clinical isolates of the microorganism for which genetic variations are correlated to antimicrobial resistance and/or susceptibility; and determining the genetic variations in the at least one clinical isolate of the microorganism associated with antimicrobial drug resistance.

In the fourteenth aspect, the first pan genome is preferably constructed predominantly using a plurality of clinical isolates of the microorganism for which genetic variations are correlated to antimicrobial resistance and/or susceptibility, particularly preferably only using a plurality of clinical isolates of the microorganism for which genetic variations are correlated to antimicrobial resistance and/or susceptibility. This way the first pan-genome is correlated with antimicrobial resistance and/or susceptibility.

According to certain embodiments, the method of the fourteenth aspect further comprises a step of determining a second pan-genome using the first pan-genome as well as the genomes used for constructing it and the first data set of at least one nucleic acid sequence of the at least one clinical isolate of the microorganism when the at least one nucleic acid sequence of at least one clinical isolate of the microorganism is correlated to antimicrobial resistance and/or susceptibility, as determined by the method of the fourteenth aspect. This way it can be ensured that the resulting second pan-genome is still based on a plurality of clinical isolates of the microorganism for which genetic variations are correlated to antimicrobial resistance and/or susceptibility.

Other steps can be similar or identical as described in e.g. the fourth or fifth aspect or other related methods.

According to certain embodiments, the first pan-genome is a pan-genome of the third aspect in the fifth or fourteenth aspect.

A sixth aspect of the present invention is directed to a method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations not correlated to antimicrobial resistance and/or susceptibility by comparison to a first pan-genome to obtain a third data set of genetic variants, and analyzing the at least one nucleic acid sequence of the first data set for genetic variations correlated to anti-microbial resistance and/or susceptibility by comparison to the first pan-genome, wherein the first pan-genome is obtained at least partially using a plurality of clinical isolates of the microorganism for which genetic variations are correlated to antimicrobial resistance and/or susceptibility;
providing a second data set of antimicrobial drug resistance and/or susceptibility of the at least one clinical isolate of the microorganism;
correlating the third data set with the second data set and statistically analyzing the correlation;
determining the genetic variations in the at least one clinical isolate of the microorganism associated with antimicrobial drug resistance; and
determining a second pan-genome using the first pan-genome as well as the genomes used for constructing it and the first data set of at least one nucleic acid sequence of the at least one clinical isolate of the microorganism.

Again, according to certain embodiments, the second pan-genome can be used for analyzing the at least one nucleic acid sequence of the first data set for genetic variations by comparison to the second pan-genome to obtain a third data set of genetic variants, which then can again be correlated, as e.g. also in the fourth aspect, thus improving the determination of the genetic variations. Particularly, genetic variations can be identified more accurately since also genes which are unique to the first data set are then part of the second pan genome, which reduces the noise during the variant calling.

Other steps can be similar or identical as described in e.g. the fourth and/or fifth aspect or other related methods.

A further, fifteenth, aspect of the present invention is directed to a method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations not correlated to antimicrobial resistance and/or susceptibility by comparison to a first pan-genome to obtain a third data set of genetic variants, and analyzing the at least one nucleic acid sequence of the first data set for genetic variations correlated to anti-microbial resistance and/or susceptibility by comparison to the first pan-genome, wherein the first pan-genome is obtained at least partially using a plurality of clinical isolates of the microorganism for which genetic variations are correlated to antimicrobial resistance and/or susceptibility;
providing a second data set of antimicrobial drug resistance and/or susceptibility of the at least one clinical isolate of the microorganism;
correlating the third data set with the second data set and statistically analyzing the correlation; and
determining the genetic variations in the at least one clinical isolate of the microorganism associated with antimicrobial drug resistance.

In the fifteenth aspect, the whole information of a first-pan-genome which contains markers for antimicrobial resistance and/or susceptibility is taken into account.

Other steps can be similar or identical as described in e.g. the fourth, fifth, sixth and/or fourteenth aspect or other related methods.

According to certain embodiments, the first pan-genome is a pan-genome of the third aspect in the sixth or fifteenth aspect.

According to certain embodiments of the fourth, fifth or sixth, or fourteenth or fifteenth, aspect, the first pan-genome is for *Escherichia coli* and is obtained using at least 150 clinical isolates, preferably at least 250 clinical isolates; or the first pan-genome is for *Acinetobacter baumanii* and is obtained using at least 150 clinical isolates, preferably at least 180 clinical isolates; or the first pan-genome is for *Citrobacter koseri* and is obtained using at least 35 clinical isolates, preferably at least 55 clinical isolates; or the first pan-genome is for *Enterobacter aerogenes* and is obtained using at least 120 clinical isolates, preferably at least 160 clinical isolates; or the first pan-genome is for *Enterobacter cloacae* and is obtained using at least 100 clinical isolates, preferably at least 150 clinical isolates; or the first pan-genome is for *Klebsiella oxytoca* and is obtained using at least 90 clinical isolates, preferably at least 120 clinical isolates; or the first pan-genome is for *Klebsiella pneumoniae* and is obtained using at least 160 clinical isolates, preferably at least 250 clinical isolates; or the first pan-genome is for *Morganella morganii* and is obtained using at least 70 clinical isolates, preferably at least 120 clinical isolates; or the first pan-genome is for *Proteus mirabilis* and is obtained using at least 120 clinical isolates, preferably at least 140 clinical isolates; or the first pan-genome is for *Pseudomonas aeruginosa* and is obtained using at least 100 clinical isolates, preferably at least 140 clinical isolates; or the first pan-genome is for *Salmonella enterica* and is obtained using at least 130 clinical isolates, preferably at least 190 clinical isolates; or the first pan-genome is for *Staphylococcus aureus* and is obtained using at least 180 clinical isolates, preferably at least 260 clinical isolates; or the first pan-genome is for *Serratia marcescens* and is obtained using at least 230 clinical isolates, preferably at least 330 clinical isolates; or the first pan-genome is for *Shigella boydii* and is obtained using at least 60 clinical isolates, preferably at least 75 clinical isolates; or the first pan-genome is for *Shigella flexneri* and is obtained using at least 50 clinical isolates, preferably at least 80 clinical isolates; or the first pan-genome is for *Shigella sonnei* and is obtained using at least 60 clinical isolates, preferably at least 80 clinical isolates; or the first pan-genome is for *Stenotrophomonas maltophilia* and is obtained using at least 125 clinical isolates, preferably at least 240 clinical isolates.

In the methods of the fourth, fifth or sixth, or fourteenth or fifteenth, aspect, as well as the other methods of the invention, the first data set of at least one nucleic acid sequence of the at least one clinical isolate of the microorganism can be provided or obtained in any way, preferably non-invasive, and can be e.g. provided from *in vitro* samples.

According to certain aspects, at least two genetic variations of the nucleic acid sequence(s) can be determined, leading to improved results compared to determining only one genetic variation. It is not excluded, though, that only one genetic variation is determined.

According to certain embodiments, more than one, e.g. two, three, four, five, six, seven, eight, nine, ten, eleven, twelve, or more, genetic variations are determined. According to certain embodiments, the correlation and statistical analysis can encompass a technique wherein all genetic variations are taken into account and then optimized for obtaining the genetic variations in the nucleic acid sequences of the microorganism associated with antimicrobial drug, e.g. antibiotic, resistance which have improved statistical relevance, e.g. can obtain a higher probability to be association with antimicrobial drug, e.g. antibiotic, resistance. According to certain embodiments, a statistical analysis can be carried out using a classification approach/method like a decision tree, random forest, neural network, bayesian classification, support vector machine, etc. wherein at first the presence of a single nucleotide polymorphism and/or structural variation is determined, e.g. a decision tree, wherein in the decision tree at first the presence of a single nucleotide polymorphism and/or structural variation is determined. A classification approach can be suitably selected and applied, e.g. a decision tree can be generated using known methods, e.g. within the scope of the statistical analysis, and is otherwise not particularly restricted. According to certain embodiments, a resistance in the microorganism can be determined using a decision tree, corresponding to a statistical analysis.

According to certain embodiments, the obtaining or providing of a first data set of at least one nucleic acid sequence of the at least one clinical isolate of the microorganism can comprise the following:
A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of at least one microorganism of interest, particularly a bacterial microorganism. For example, sequencing can be carried out using polymerase chain reaction (PCR), particularly multiplex PCR, or high throughput sequencing or next generation sequencing, preferably using high-throughput sequencing. For sequencing, preferably an *in vitro* sample is used.

The obtaining or providing of nucleic acid sequences thereby can include obtaining or providing chromosomal nucleic acid sequences as well as plasmid nucleic acid sequences, particularly the whole nucleic acid sequences of the microorganism, e.g. bacterial microorganism.

The data obtained by the sequencing can be in any format, and can then be used to identify the nucleic acids of the microorganism to be identified, by known methods, e.g. fingerprinting methods, comparing nucleic acid sequences, e.g. genomes. When forming the third data set of nucleic acid sequences, e.g. genes, for a microorganism, additional data from other sources, e.g. the vertebrate, can be discarded. For the present method, also the raw data can be used assembled, at least in part. Thus, according to certain embodiments, at least a part of the nucleic acid, e.g. gene, sequences of the first data set can be assembled, wherein assembly can be carried out by any known method and is not particularly limited.

For constructing pan-genomes, also assembled data can be used, e.g. nucleic acid sequence data obtained by sequencing of samples can be assembled and then calculated, e.g. using Roary (Rapid large-scale prokaryote pan genome analysis (Bioinformatics 2015 Nov 15;31(22): 3691-3. doi: 10.1093/bioinformatics/btv421. Epub 2015 Jul 20. Page AJ, Cummins CA, et al.).

Pan-genomes offer the advantage that they contain chromosomal nucleic acid sequences as well as plasmid nucleic acid sequences, i.e. a comparison to the pan-genome enables a fast and complete analysis of the first data set for genetic variations. Further, a pan-genome also allows for a more complete analysis for genetic variations as the pan-genome also allows for variation in gene content among closely related strains.

Also, according to certain embodiments, it is useful in genome-wide association studies to reference the points of interest, e.g. structural variations and/or SNPs, to one constant reference for enhanced standardization, which can be a stable pan-genome, e.g. as provided by the present invention.

In the present method, nucleic acid, e.g. gene, but also noncoding, sequence of the first data set can also be assembled, at least in part, according to certain embodiments with known methods, e.g. by de-novo assembly or mapping assembly, reference guided assembly. The sequence assembly is not particularly limited, and any known nucleic acid sequence assembler can be used, e.g. based on Sanger, 454, Solexa, Illumina, SOLid technologies, etc., as well as hybrids/mixtures thereof.

According to certain embodiments, the data of nucleic acids of different origin than the microorganism of interest, e.g. a bacterial microorganism, can be removed after the nucleic acids of interest are identified, e.g. by filtering the data out. Such data can e.g. include nucleic acids of a patient, e.g. the vertebrate, e.g. human, and/or other microorganisms, etc. This can be done by e.g. computational subtraction, as developed by Meyerson et al. 2002. For this, also aligning to the nucleic acid sequences, e.g. genome, of the vertebrate, etc., is possible. For aligning, several alignment-tools are available. This way the original data amount from the sample can be drastically reduced.

After such removal of "excess" data, the possible obtaining of the third data set can be carried out for the microorganism, e.g. a bacterial microorganism, as described above and below.

Regarding culturing methods for obtaining the second data set, which are nor limited, (a) sample(s) of microorganism(s) can be e.g. cultured overnight. On the next day individual colonies can be used for identification of organisms, either by culturing or using mass spectroscopy. Based on the identity of organisms new plates containing increasing concentration of antibiotics used for the treatment of these organisms are inoculated and grown for additional 12 - 24 hours. The lowest drug concentration which inhibits growth (minimal inhibitory concentration - MIC) can be used to determine susceptibility/resistance for tested antibiotics.

Also, resistance testing can be carried out by determining e.g. known resistance genes in the different isolates, like in case of methicillin resistant Staphylococcus aureus (MRSA) and methicillin susceptible Staphylococcus aureus (MSSA). For determining resistances, respectively susceptibility, the data from culturing methods and/or from determining known resistance genes, as well as data obtained in different ways, e.g. based on mass spectrometry (possibly also in connection with culturing) can be used.

Correlation of the genetic variations with antimicrobial drug, e.g. antibiotic, resistance can be carried out in a usual way and is not particularly limited. For example, resistances can be correlated to structural variations and/or SNPs in the whole nucleic acid sequence(s) of the respective microorganism or only parts thereof, for example only coding parts of the nucleic acid sequence(s). In some cases even only genetic variations, i.e. structural variations and/or SNPS in nucleic acid molecules with certain nucleic acid sequences, e.g. genes, e.g. certain genes, or certain mutations in nucleic acid molecules with certain nucleic acid sequences, e.g. genes, can be determined. After correlation, statistical analysis can be carried out, e.g. when using a plurality of clinical isolates.

According to certain embodiments, the data of the first data set can be filtered prior to a possible annotation to the first pan-genome and the possible correlation with the resistance/susceptibility data, e.g. when determining structural variations.

For example, to reduce the number of similar annotations for genetic variations, e.g. for structural variations and/or SNPs, they can be filtered and aggregated by one or more of the following:
- Only annotations for which the considered genetic variation, e.g. structural variation and/or SNP, lies on a protein can be kept and the further data discarded
- Only annotations which do not contain "hypothetical proteins" can be kept
- Annotations can be sorted by identification number (ID) for genetic variations, e.g. for SNPs and/or structural variation, and nucleic acid sequence, e.g. gene product
- For a unique pair of IDs and nucleic acid sequences, e.g. gene products, only the first annotation can be kept, e.g. in case of multiple nucleic acid sequences, e.g. coding certain genes, in a genome

Also, according to certain embodiments, the following genetic variations, e.g. structural variations and/or SNPs, can be excluded:
1. Constant features and phenotypes (same value or only NA (not available)) can be removed (e.g. centroids present in all samples or phenotypes with the result "resistant" for all samples)
2. Almost constant features and phenotypes can also be removed, e.g. features whose most frequent value was in >=95% of all samples, ignoring NA values, can be removed (e.g. a centroid is present in >=95% of all samples)
   ∘ Also phenotypes whose most frequent value was in >=90% of all samples, ignoring NA values, can be removed (e.g. >=90% of all samples are resistant)
3. In addition, only drugs with non-missing data for at least 10% of the samples can be kept.
4. Genetic variations, e.g. SNPs and/or structural variations, without any annotation or mutations, e.g. SNPs and/or structural variations whose all annotations contain flag "synonymous", can be removed so that only mutations, e.g. SNPs and/or structural variations, with at least one non-synonymous annotation, e.g. a non-synonymous coding, are considered.

A possible statistical analysis is not particularly limited and can be suitably carried out.

For statistical analysis, e.g. Fisher's exact two-sided test can be applied with subsequent p-value adjustment over all phenotypes together using e.g. familywise error rate (FWER) or FDR (false discovery rate) and p-value threshold of 0.01 (corresponding to 10⁻², respectively 1e-2). Additionally, 10 permutation tests can be performed by permuting each phenotype separately and applying Fisher's exact test, e.g. to the centroid presence matrix and permuted phenotypes. Regarding centroids, the results then can be further filtered by centroid annotation, i.e.
1. Centroids without a gene product name can optionally be removed
2. Centroids whose gene product name contains "putative", "predicted" or "hypothetical" can be removed
3. If there are centroids with same gene product name and gene symbol than only the first one can be kept
4. Centroids without GeneBank accession can be removed

Other statistical analysis can also be carried out alternatively or in addition, though.

The construction of a pan-genome is not particularly limited and can be done using known methods. For example, assembled data, e.g. of several individual samples, of the microorganism, can be used for constructing a pan-genome, e.g. nucleic acid sequence data obtained by sequencing of the samples can be assembled and then calculated, e.g. using Roary (Rapid large-scale prokaryote pan genome analysis (Bioinformatics 2015 Nov 15;31(22): 3691-3. doi: 10.1093/bioinformatics/btv421. Epub 2015 Jul 20. Page AJ, Cummins CA, et al.).

When referring to a second data set, wherein the second data set e.g. comprises, respectively is, a set of antimicrobial drug, e.g. antibiotic, resistances of a plurality of clinical isolates, this can, within the scope of the invention, also refer to a self-learning data base that, whenever a new sample is analyzed, can take this sample into the second data set and thus expand its data base. The second data set thus does not have to be static and can be expanded, either by external input or by incorporating new data due to self-learning. The same applies, where applicable, to the first data set.

According to certain embodiments of the present methods - as above, the second data set can be provided by culturing the clinical isolates of the microorganism on suitable plates, e.g. agar plates, provided with antimicrobial drugs, e.g. antibiotics, at different concentrations, and the second data can be obtained by taking the minimal concentration of the plates that inhibits growth of the respective microorganism.

According to certain embodiments the antimicrobial drug, e.g. antibiotic drug, is selected from the group consisting of β-lactams, β-lactam inhibitors, quinolones and derivatives thereof, e.g. fluoroquinolones, aminoglycosides, glycopeptides, lincosamides, macrolides, nitrofuranes, oxazolidinones, polyketides, respectively tetracyclines, and folate synthesis inhibitors, e.g. benzene derived/sulfonamide antibiotics. According to certain embodiments, the antimicrobial drug, e.g. antibiotic drug, is selected from the group consisting of Amoxicillin/K Clavulanate (AUG), Ampicillin (AM), Aztreonam (AZT), Cefazolin (CFZ), Cefepime (CPE), Cefotaxime (CFT), Ceftazidime (CAZ), Ceftriaxone (CAX), Cefuroxime (CRM), Cephalotin (CF), Ciprofloxacin (CP), Ertapenem (ETP), Gentamicin (GM), Imipenem (IMP), Levofloxacin (LVX), Meropenem (MER), Piperacillin/Tazobactam (P/T), Ampicillin/Sulbactam (A/S), Tetracycline (TE), Tobramycin (TO), and Trimethoprim/Sulfamethoxazole (T/S). According to certain embodiments, the microorganism is a Gram-positive or a Gram-negative bacteria, e.g. a Gram-negative bacteria.

In the methods of the invention, the resistance of the microorganism, particularly the bacterial microorganism, to one or more antimicrobial, e.g. antibiotic, drugs can be determined.

According to certain embodiments, the resistance of a microorganism, particularly bacterial microorganism, against 1, 2, 3, 4, 5, 6, 7, 8, 9, 10, 11, 12, 13, 14, 15 or 16, 17, 18, 19, 20, 21 or more antibiotic drugs is determined. According to certain embodiments, the resistance of a microorganism, particularly bacterial microorganism, against 1, 2, 3, 4, 5, 6, 7, 8, 9, 10, 11, 12, 13, 14, 15 or 16, 17, 18, 19, 20 or 21 antibiotic drugs is determined.

A seventh aspect of the present invention relates to a pan-genome, wherein the pan-genome is for *Escherichia coli* and is obtained using at least 150 clinical isolates, preferably at least 250 clinical isolates; or wherein the pan-genome is for *Acinetobacter baumanii* and is obtained using at least 150 clinical isolates, preferably at least 180 clinical isolates; or wherein the pan-genome is for *Citrobacter koseri* and is obtained using at least 35 clinical isolates, preferably at least 55 clinical isolates; or wherein the pan-genome is for *Enterobacter aerogenes* and is obtained using at least 120 clinical isolates, preferably at least 160 clinical isolates; or wherein the pan-genome is for *Enterobacter cloacae* and is obtained using at least 100 clinical isolates, preferably at least 150 clinical isolates; or wherein the pan-genome is for *Klebsiella oxytoca* and is obtained using at least 90 clinical isolates, preferably at least 120 clinical isolates; or wherein the pan-genome is for *Klebsiella pneumoniae* and is obtained using at least 160 clinical isolates, preferably at least 250 clinical isolates; or wherein the pan-genome is for *Morganella morganii* and is obtained using at least 70 clinical isolates, preferably at least 120 clinical isolates; or wherein the pan-genome is for *Proteus mirabilis* and is obtained using at least 120 clinical isolates, preferably at least 140 clinical isolates; or wherein the pan-genome is for *Pseudomonas aeruginosa* and is obtained using at least 100 clinical isolates, preferably at least 140 clinical isolates; or wherein the pan-genome is for *Salmonella enterica* and is obtained using at least 130 clinical isolates, preferably at least 190 clinical isolates; or wherein the pan-genome is for *Staphylococcus aureus* and is obtained using at least 180 clinical isolates, preferably at least 260 clinical isolates; or wherein the pan-genome is for *Serratia marcescens* and is obtained using at least 230 clinical isolates, preferably at least 330 clinical isolates; or wherein the pan-genome is for *Shigella boydii* and is obtained using at least 60 clinical isolates, preferably at least 75 clinical isolates; or wherein the pan-genome is for *Shigella flexneri* and is obtained using at least 50 clinical isolates, preferably at least 80 clinical isolates; or wherein the pan-genome is for *Shigella sonnei* and is obtained using at least 60 clinical isolates, preferably at least 80 clinical isolates; or wherein the pan-genome is for *Stenotrophomonas maltophilia* and is obtained using at least 125 clinical isolates, preferably at least 240 clinical isolates.

Also disclosed is - in a sixteenth aspect - a data bank comprising at least one pan-genome of the seventh aspect, preferably comprising 2, 3, 4, 5, 6, 7, 8, 9, 10, 11, 12, 13, 14, 15, 16, or 17 of the pan-genomes of the seventh aspect.

In an eight aspect, the use of the pan-genome of the third or seventh aspect for the determination of bacterial resistance in a sample from a patient is disclosed. Of course also other uses are possible, as discussed above.

A ninth aspect of the present invention relates to a, e.g. diagnostic, method of determining an infection of a patient with a microorganism, comprising the steps of:
a) obtaining or providing a sample containing or suspected of containing a microorganism from the patient;
b) determining the presence of at least one genetic variation in at least one genetic sequence of the microorganism, as determined by the method of any one of the fourth, fifth, sixth, fourteenth or fifteenth aspect of the present invention, wherein the presence of said at least one genetic variation is indicative of an infection with an antimicrobial drug resistant microorganism in said patient.

According to certain embodiments, the microorganism is a bacterial microorganism potentially resistant to antimicrobial drug treatment.

In the infection of a patient with a microorganism, preferably a bacterial microorganism, e.g. one or more of Acinetobacter, Citrobacter, Escherichia, Enterobacter, Klebsiella, Morganella, Proteus, Pseudomonas, Salmonella, Serratia, Shigella, Stenotrophomonas and/or Staphylococcus species, e.g. *Escherichia coli, Acinetobacter baumanii, Citrobacter koseri, Enterobacter aerogenes, Enterobacter clo*acae, *Klebsiella oxytoca, Klebsiella pneumoniae, Morganella morganii, Proteus mirabilis, Pseudomonas aeruginosa, Salmonella enterica, Staphylococcus aureus, Serratia marcescens, Shigella boydii, Shigella flexneri, Shigella sonnei,* and *Stenotrophomonas* maltophilia, the microorganism is preferably potentially resistant to antimicrobial drug treatment, which herein means an infection of a patient with a microorganism, preferably a bacterial microorganism, particularly one as noted above, wherein it is unclear if the microorganism, preferably bacterial microorganism, is susceptible to treatment with a specific antimicrobial drug or if it is resistant to the antimicrobial drug.

With this method, any genetic variations / mutations in the nucleic acid sequences of a microorganism, e.g. bacterial microorganism, e.g. a clinical isolate with an unknown strain of the microorganism, particularly bacterial microorganism, correlated with antimicrobial drug, e.g. antibiotic, resistance can be determined and a thorough antimicrobial drug, e.g. antibiotic, resistance profile can be established comprising structural variations as well as SNPs.

The different steps can herein be carried out as described with regard to the further related methods of the present invention.

According to this aspect, an infection with a microorganism, particularly a bacterial microorganism, in a patient can be determined using sequencing methods, and a resistance to antimicrobial drugs, e.g. antibiotics, of the microorganism can be determined in a short amount of time compared to conventional methods.

In a tenth aspect the present invention relates to a method of selecting a treatment of a patient suffering from an infection with a potentially resistant microorganism, comprising the steps of:
a) obtaining or providing a sample containing or suspected of containing a microorganism from the patient;
b) determining the presence of at least one genetic variation in at least one genetic sequence of the microorganism, as determined by the method of any one of the fourth, fifth, sixth, fourteenth or fifteenth aspect of the present invention, wherein the presence of said at least one genetic variation is indicative of an infection with an antimicrobial drug resistant microorganism in said patient;
c) identifying said at least one or more antimicrobial drugs; and
d) selecting one or more antimicrobial drugs different from the ones identified in step c) and being suitable for the treatment of the infection with the microorganism.

This method can be carried out similarly to the one in the ninth aspect of the invention and enables a fast way to select a suitable treatment with antibiotics for any infection with an unknown microorganism, particularly bacterial microorganism.

According to certain embodiments, genetic variations, e.g. structural variations and/or SNPs, in at least two, three, four, five, six, seven, eight, nine, ten, eleven, twelve, or more positions, respectively sequences, are determined in any of the methods of the present invention, e.g. in at least two positions, respectively sequences, or in at least three positions, respectively sequences, in the chromosomal nucleic acid sequences and/or the plasmid nucleic acid sequences. Instead of testing only single positions and/or sequences, the combination of several genetic variations, e.g. variant positions and/or sequences, can improve the prediction accuracy and further reduce false positive findings that are influenced by other factors. Therefore, it is in particular preferred to determine the presence of structural variations and/or SNPs in 2, 3, 4, 5, 6, 7, 8, 9, 10, 11, 12 (or more) sequences.

The identification of the at least one or more antimicrobial, e.g. antibiotic, drug in step c) can be based on the results obtained in step b) and corresponds to the antimicrobial, e.g. antibiotic, drug(s) that correlate(s) with the genetic variations. Once these antimicrobial drugs, e.g. antibiotics, are ruled out, the remaining antimicrobial drugs, e.g. antibiotic drugs/antibiotics, can be selected in step d) as being suitable for treatment.

According to certain embodiments in the ninth or tenth aspect, step b) is carried out using a classification approach/method like a decision tree, random forest, neural network, bayesian classification, support vector machine, etc. wherein at first the presence of a genetic variation is determined, e.g. a decision tree, wherein in the decision tree at first the presence of a single nucleotide polymorphism and/or structural variation is determined. A classification approach can be suitably selected and applied, e.g. a decision tree can be generated using known methods, e.g. within the scope of the statistical analysis, and is otherwise not particularly restricted. According to certain embodiments, a resistance in the microorganism can be determined using a decision tree, corresponding to a statistical analysis. This way the diagnosis of a resistant microorganism, e.g. bacterial microorganism, can be optimized.

According to certain embodiments, determining the nucleic acid sequence information or the presence of a genetic variation in the present methods comprises using a next generation sequencing or high throughput sequencing method, e.g. as mentioned above.

According to certain embodiments of any of the methods of the present invention the genetic variation is selected from at least one of structural variations of the nucleic acid sequences comprising at least a change in the nucleic acid sequence comprising more than one base, and single nucleotide polymorphisms (SNPs).

An eleventh aspect of the present invention is directed to a method of treating a patient suffering from an antimicrobial drug, e.g. antibiotic, resistant infection with a microorganism, preferably a bacterial microorganism, comprising the steps of:
a) obtaining or providing a sample containing or suspected of containing a microorganism from the patient;
b) determining the presence of at least one genetic variation in at least one genetic sequence of the microorganism, as determined by the method of any one of the fourth, fifth, sixth, fourteenth or fifteenth aspect of the present invention, wherein the presence of said at least one genetic variation is indicative of an infection with an antimicrobial drug resistant microorganism in said patient;
c) identifying said at least one or more antimicrobial drugs; and
d) selecting one or more antimicrobial drugs different from the ones identified in step c) and being suitable for the treatment of the infection with the microorganism; and
e) treating the patient with said one or more antimicrobial, e.g. antibiotic, drugs.

Herein, steps a) to d) can be carried out as described with respect to the fourth aspect. Step e) can be sufficiently carried out without being restricted and can be done e.g. non-invasively.

In a twelfth aspect one or more computer program products comprising computer executable instructions which, when executed, perform a method according to any one of the fourth, fifth, sixth, ninth, tenth, fourteenth or fifteenth aspect of the present invention, are disclosed.

In certain embodiments the computer program product is one on which program commands or program codes of a computer program for executing said method are stored. According to certain embodiments the computer program product is a storage medium. As noted above, the computer program products of the present invention can be self-learning, e.g. with respect to the first and second data sets and/or the pan-genome.

In order to obtain the best possible information from the highly complex genetic data and develop an optimum model for diagnostic and therapeutical uses as well as the methods of the present invention - which can be applied stably in clinical routine - a thorough in silico analysis can be necessary. The proposed principle is based on a combination of different approaches, e.g. optional assembly of the nucleic acid, e.g. gene, sequences and/or genome of the microorganisms, at least in part and alignment of the sequence data of the clinical isolate to be determined with one or more pan-genomes, and possibly correlation of genetic variations found in every sample, e.g. from each patient, respectively an unknown clinical isolate, with all drugs, e.g. antibiotics, or only one or some of them, and search for genetic variations for one or several drugs and in one or several strains.

Using the above steps a list of genetic variations with regard to one or more pan-genomes is generated. This can be stored in databases, and statistical models can be derived from the databases. The statistical models can be based on at least one or more genetic variations. Statistical models that can be trained can be combined from genetic variations and/or nucleic acid sequences. Examples of algorithms that can produce such models are association Rules, Support Vector Machines, Decision Trees, Decision Forests, Discriminant-Analysis, Cluster-Methods, and many more.

The goal of the training is to allow a reproducible, standardized application during routine procedures.

For this, for example, nucleic acid, e.g. gene, sequences or parts thereof can be sequenced from a patient to be diagnosed. Afterwards, core characteristics can be derived from the sequence data which can be used to predict resistance.

The corresponding characteristics can be used as input for the statistical model and thus enable a prognosis for new patients. Not only the information regarding all resistances of all microorganisms, against all or only some or one drugs, e.g. antibiotics, can be integrated in a computer decision support tool, but also corresponding directives (e.g. EUCAST) so that only treatment proposals are made that are in line with the directives.

A thirteenth aspect of the present invention relates to the use of the computer program product according to the twelfth aspect, e.g. for determining genetic variations in the nucleic acid sequences of a microorganism for a clinical isolate of the microorganism in the fourth, fifth, sixth, fourteenth of fifteenth aspect of the invention and/or for use in the method of the ninth aspect of the invention and/or for selecting a treatment in the tenth aspect of the present invention and/or in the method of the eleventh aspect of the present invention.

### Examples

The present invention will now be described in detail with reference to several examples thereof. However, these examples are illustrative and do not limit the scope of the invention.

Pan-genomes were obtained by the method described below for several bacterial species.

For constructing pan-genomes, the following strains/species were considered, and the number of individual samples used in each case was as given in Table 1.

**Table 1: Number of samples used for each species**

| **Species** | **Number of samples** |
|---|---|
| *Acinetobacter baumannii* | 435 |
| *Citrobacter koseri* | 111 |
| *Escherichia coli* | 941 |
| *Enterobacter aerogenes* | 325 |
| *Enterobacter cloacae* | 549 |
| *Klebsiella oxytoca* | 310 |
| *Klebsiella pneumoniae* | 1101 |
| *Morganella morganii* | 286 |
| *Proteus mirabilis* | 457 |
| *Pseudomonas aeruginosa* | 1132 |
| *Salmonella enterica* | 633 |
| *Staphylococcus aureus* | 958 |
| *Serratia marcescens* | 510 |
| *Shigella boydii* | 78 |
| *Shigella flexneri* | 98 |
| *Shigella sonnei* | 226 |
| *Stenotrophomonas maltophilia* | 501 |

Nucleic acid sequencing was carried out in addition to classical antimicrobial susceptibility testing of the same isolates. This allowed performing genome wide correlation studies to find genetic variants (e.g. point mutations, small insertions and deletion, larger structural variants, plasmid copy number gains, gene dosage effects) in the nucleic acid sequences that are significantly correlated to the resistance against one or several drugs. The approach also allows for comparing the relevant sites in the genome to each other.

For determining genetic variants that can be in the chromosomal nucleic acid sequences as well as in plasmid nucleic acid sequences, pan-genomes were constructed, as these cover both the chromosome and the plasmids. For constructing the pan-genomes, the data obtained by sequencing of the samples, as detailed below, were assembled and calculated using Roary (Rapid large-scale prokaryote pan genome analysis (Bioinformatics 2015 Nov 15;31(22): 3691-3. doi: 10.1093/bioinformatics/btv421. Epub 2015 Jul 20. Page AJ, Cummins CA, et al.). *De novo* assemblies were constructed using SPAdes (version 3.6.2, Bankevich A, Nurk S, Antipov D, et al. SPAdes: A New Genome Assembly Algorithm and Its Applications to Single-Cell Sequencing. Journal of Computational Biology. 2012;19(5):455-477. doi:10.1089/cmb.2012.0021) with parameters -m 254 -k 21,33,55 --careful -1 fp.fastq.gz -2 rp.fastq.gz where fp.fastq.gz and rp.fastq.gz are trimmed reads processed using Trimmomatic (version 0.35, http://www.ncbi.nlm.nih.gov/pmc/articles/PMC4103590) with parameters PE <forward reads> <reverse reads> <output files> ILLUMINACLIP:NexteraPE-PE:1:50:30 LEADING:3 TRAILING:3 SLIDINGWINDOW:4:15 MINLEN:36. To determine the quality of the assemblies we ran QUAST (version 3.2) with minimal length threshold of 200 bp. Assemblies were annotated using Prokka (1.11) using parameters --force --outdir <output dir>prefix <sample ID> --locustag <sample ID> --centre ICMBgram neg --mincontiglen 200 <scaffolds fasta>. Furthermore, taxonomy of the samples was determined using Kraken (version 0.10.4-beta, finished bacterial and viral genomes from NCBI RefSeq downloaded on 2015.01.13) based on raw NGS reads using k-mer length of 31 and default parameters. Samples were grouped by the assigned species taxon (at least 50 samples per group) and filtered by the assembly quality (max. L50 = 200, max. #contigs(scaffolds) = 1000, min. N50 = 5000, "RefSeq microbial genomes database: new representation and annotation strategy", http://www.ncbi.nlm.nih.gov/pubmed/24316578) and taxonomic assignment quality (Shigella species: min. sens. = 0%, min. prec. = 60%, max. unclassified = 30%; other species: min. sens. = 50%, min. prec. = 75%, max. unclassified = 30% where sens. = sensitivity = (# reads assigned to species taxon) / (# processed reads) and prec. = precision = (# reads assigned to species taxon) / (# reads assigned to any species taxon).

The following pan-genomes were obtained:
*Escherichia coli:* SEQ ID NO 1 - 41296,
*Acinetobacter baumanii:* SEQ ID NO 41297 - 71315,
*Citrobacter koseri:* SEQ ID NO 71316 - 86840,
*Enterobacter aerogenes:* SEQ ID NO 86841 - 113784,
*Enterobacter cloacae:* SEQ ID NO 113785 - 171921,
*Klebsiella oxytoca:* SEQ ID NO 171922 - 209528,
*Klebsiella pneumoniae:* SEQ ID NO 209529 - 273591,
*Morganella morganii:* SEQ ID NO 273592 - 293457,
*Proteus mirabilis:* SEQ ID NO 293458 - 318000,
*Pseudomonas aeruginosa:* SEQ ID NO 318001 - 368053,
*Salmonella enterica:* SEQ ID NO 368054 - 404774,
*Staphylococcus aureus:* SEQ ID NO 404775 - 421990,
*Serratia marcescens:* SEQ ID NO 421991 - 457642,
*Shigella boydii:* SEQ ID NO 457643 - 470165,
*Shigella flexneri:* SEQ ID NO 470166 - 481587,
*Shigella sonnei:* SEQ ID NO 481588 - 492644,
*Stenotrophomonas maltophilia:* SEQ ID NO 492645 - 537429

Using the pan-genomes and the data obtained for antibiotic resistance (as described below), genetic variants were determined within the pan-genome that are correlated to antibiotic resistance.

In the present approach the different sources of genetic resistance regarding structural variances as well as the different ways of how bacteria can become resistant were covered. By measuring clinical isolates collected in a broad geographical area and across a broad time span of three decades a complete picture going far beyond the rather artificial step of laboratory generated resistance mechanisms was tried to be generated.

To this end, a set of 21 clinically relevant antimicrobial agents with 5 different modes of action was put together, and the minimally inhibitory concentration (MIC) of the 21 drugs for the isolates was measured.

The detailed procedure is given in the following:

### Bacterial Strains

The inventors selected strains from the microbiology strain collection at Siemens Healthcare Diagnostics (West Sacramento, CA) for susceptibility testing and nucleic acid sequencing.

Antimicrobial Susceptibility Testing (AST) Panels Frozen reference AST panels were prepared following Clinical Laboratory Standards Institute (CLSI) recommendations. The following antimicrobial agents (with µg/ml concentrations shown in parentheses) were included in the panels: Amoxicillin/K Clavulanate (0.5/0.25-64/32), Ampicillin (0.25-128), Ampicillin/Sulbactam (0.5/0.25-64/32), Aztreonam (0.25-64), Cefazolin (0.5-32), Cefepime (0.25-64), Cefotaxime (0.25-128), Ceftazidime (0.25-64), Ceftriaxone (0.25-128), Cefuroxime (1-64), Cephalothin (1-64), Ciprofloxacin (0.015-8), Ertepenem (0.12-32), Gentamicin (0.12-32), Imipenem (0.25-32), Levofloxacin (0.25-16), Meropenem (0.12-32), Piperacillin/Tazobactam (0.25/4-256/4), Tetracycline (0.5-64), Tobramycin (0.12-32), and Trimethoprim/Sulfamethoxazole (0.25/4.7-32/608). Prior to use with clinical isolates, AST panels were tested with QC strains. AST panels were considered acceptable for testing with clinical isolates when the QC results met QC ranges described by CLSI16.

### Inoculum Preparation

Isolates were cultured on trypticase soy agar with 5% sheep blood (BBL, Cockeysville, Md.) and incubated in ambient air at 35±1°C for 18-24 h. Isolated colonies (4-5 large colonies or 5-10 small colonies) were transferred to a 3 ml Sterile Inoculum Water (Siemens) and emulsified to a final turbidity of a 0.5 McFarland standard. 2 ml of this suspension was added to 25 ml Inoculum Water with Pluronic-F (Siemens). Using the Inoculator (Siemens) specific for frozen AST panels, 5 µl of the cell suspension was transferred to each well of the AST panel. The inoculated AST panels were incubated in ambient air at 35±1°C for 16-20 h. Panel results were read visually, and minimal inhibitory concentrations (MIC) were determined.

### DNA extraction

Four streaks of each Gram-negative bacterial isolate cultured on trypticase soy agar containing 5% sheep blood and cell suspensions were made in sterile 1.5 ml collection tubes containing 50 µl Nuclease-Free Water (AM9930, Life Technologies). Bacterial isolate samples were stored at -20 °C until nucleic acid extraction. The Tissue Preparation System (TPS) (096D0382-02_01_B, Siemens) and the VERSANT® Tissue Preparation Reagents (TPR) kit (10632404B, Siemens) were used to extract DNA from these bacterial isolates. Prior to extraction, the bacterial isolates were thawed at room temperature and were pelleted at 2000 G for 5 seconds. The DNA extraction protocol DNAext was used for complete total nucleic acid extraction of 48 isolate samples and eluates, 50 µl each, in 4 hours. The total nucleic acid eluates were then transferred into 96-Well qPCR Detection Plates (401341, Agilent Technologies) for RNase A digestion, DNA quantitation, and plate DNA concentration standardization processes. RNase A (AM2271, Life Technologies) which was diluted in nuclease-free water following manufacturer's instructions was added to 50 µl of the total nucleic acid eluate for a final working concentration of 20 µg/ml. Digestion enzyme and eluate mixture were incubated at 37°C for 30 minutes using Siemens VERSANT® Amplification and Detection instrument. DNA from the RNase digested eluate was quantitated using the Quant-iT™ PicoGreen dsDNA Assay (P11496, Life Technologies) following the assay kit instruction, and fluorescence was determined on the Siemens VERSANT® Amplification and Detection instrument. Data analysis was performed using Microsoft® Excel 2007. 25 µl of the quantitated DNA eluates were transferred into a new 96-well PCR plate for plate DNA concentration standardization prior to library preparation. Elution buffer from the TPR kit was used to adjust DNA concentration. The standardized DNA eluate plate was then stored at -80°C until library preparation.

### Next Generation Sequencing

Prior to library preparation, quality control of isolated bacterial DNA was conducted using a Qubit 2.0 Fluorometer (Qubit dsDNA BR Assay Kit, Life Technologies) and an Agilent 2200 TapeStation (Genomic DNA ScreenTape, Agilent Technologies). NGS libraries were prepared in 96 well format using NexteraXT DNA Sample Preparation Kit and NexteraXT Index Kit for 96 Indexes (Illumina) according to the manufacturer's protocol. The resulting sequencing libraries were quantified in a qPCR-based approach using the KAPA SYBR FAST qPCR MasterMix Kit (Peqlab) on a ViiA 7 real time PCR system (Life Technologies). 96 samples were pooled per lane for paired-end sequencing (2x 100bp) on Illumina Hiseq2000 or Hiseq2500 sequencers using TruSeq PE Cluster v3 and TruSeq SBS v3 sequencing chemistry (Illumina). Basic sequencing quality parameters were determined using the FastQC quality control tool for high throughput sequence data (Babraham Bioinformatics Institute).

### Mapping

Mapping was carried out against the pan-genomes given above and in the accompanying sequence protocol.

Raw paired-end sequencing data for the samples were mapped against the respective pan-genomes with BWA 0.7.12 The resulting SAM files were sorted, converted to BAM files, and PCR duplicates were marked using the Picard tools package 2.0.1 (http://picard.sourceforge.net/).

### Data analysis

Identification of genetic variations was as follows:
The Genome Analysis Toolkit (GATK) UnifiedGenotyper, 3.5 was used to call SNPs and indels for blocks of max. 200 samples (parameters: -ploidy 1 -glm BOTH -stand_call_conf 30 - stand_emit_conf 10). VCF files were combined into a single file and quality filtering for SNPs was carried out (QD < 2.0 || FS > 60.0 || MQ < 40.0 || HaplotypeScore > 13.0 || MappingQualityRankSum < -12.5 || ReadPosRankSum < -8.0) and indels (QD < 2.0 || FS > 200.0 || ReadPosRankSum < -20.0). Detected variants were annotated with SnpEff 4.2 to predict coding effects.

10 repetitions of 5-fold cross-validation were carried out. In each fold, features were selected by ranking them using a GWAS (genome-wide association study) approach with PCA (principal component analysis) adjustment. The final model was built from the most occurring features from the cross-validation. For obtaining an optimized model, decision tree analysis was carried out as follows:
Model: Decision tree from R-package rpart; maximal depth=5, default parameters, pruning (decreasing tree size to avoid over fitting; the set parameters affect the number of features in the model

### Comparative Examples

For comparison, analysis was also carried out by mapping the same samples to reference genomes from single isolates, obtained from NCBI, in addition to the mapping to the pan-genomes, and analyzing the data for correlation between genetic variations compared to the respective reference genome and antibiotic resistance, as obtained above.

For comparing the effect of using pan-genomes compared to reference genomes for determining antimicrobial resistance, six different bacterial strains of different genera, i.e. *Acinetobacter baumannii, Escherichia coli, Klebsiella oxytoca, Klebsiella pneumoniae, Proteus mirabilis, and Serratia marcescens,* were considered in the following examples to show that the effect is not limited to a particular strain or genus. Also, only SNPs were considered in the examples shown below for easier analysis, although similar effects as shown below were also obtained for examples wherein structural variants were taken into account.

Raw data were obtained from sequencing as described above. Data treatment differed from the mapping step in using reference genomes, as follows:
For the mapping to the reference genomes, mapping was furthermore also carried out against specific reference genomes that take into account only nucleic acid sequences on the chromosome and are as follows: NC_017847 as annotated at the NCBI for *Acinetobacter baumannii,* CP000948 (see also NC_010473), as annotated at the NCBI for *Escherichia coli,* NC_009648, as annotated at the NCBI for *Klebsiella pneumoniae,* NC_016612, as annotated at the NCBI for *Klebsiella oxytoca,* NC_010554 as annotated at the NCBI for *Proteus mirabilis,* and NC_020211 as annotated at the NCBI for *Serratia marcescens.*

The reference genomes were thereby selected for each bacterial species by correlating the data to all available chromosomal reference genomes at the NCBI and selecting the best suited.

Reference sequence for *Acinetobacter baumannii,* strain NC_017847 (http://www.ncbi.nlm.nih.gov/nuccore/NC_017847): LOCUS NC_017847 3964912 bp DNA circular CON 01-MAR-2015
DEFINITION Acinetobacter baumannii MDR-TJ, complete genome.
ACCESSION NC_017847 NZ_AEOE01000000 NZ_AEOE01000001 NZ_AEOE01000002 NZ_AEOE01000003 NZ_AEOE01000004
VERSION NC_017847.1 GI:387122089
DBLINK BioProject: PRJNA224116 BioSample: SAMN02603104 Assembly: GCF_000187205.2
KEYWORDS RefSeq.
SOURCE Acinetobacter baumannii MDR-TJ ORGANISM Acinetobacter baumannii MDR-TJ Bacteria; Proteobacteria; Gammaproteobacteria;
Pseudomonadales; Moraxellaceae; Acinetobacter; Acinetobacter calcoaceticus/baumannii complex.

### REFERENCE 1 (bases 1 to 3964912)

AUTHORS Huang,H., Yang,Z.L., Wu,X.M., Wang,Y., Liu,Y.J., Luo,H., Lv,X., Gan,Y.R., Song,S.D. and Gao,F.
TITLE Complete genome sequence of Acinetobacter baumannii MDR-TJ and insights into its mechanism of antibiotic resistance
JOURNAL J. Antimicrob. Chemother. 67 (12), 2825-2832 (2012) PUBMED 22952140

### REFERENCE 2 (bases 1 to 3964912)

AUTHORS Gao,F., Wang,Y., Liu,Y.J., Wu,X.M., Lv,X., Gan,Y.R., Song,S.D. and Huang,H.
TITLE Genome sequence of Acinetobacter baumannii MDR-TJ
JOURNAL J. Bacteriol. 193 (9), 2365-2366 (2011) PUBMED 21398552

### REFERENCE 3 (bases 1 to 3964912)

AUTHORS Huang,H., Yang,Z.-L., Wu,X.-M., Wang,Y., Liu,Y.-J., Luo,H., Lv,X., Gan,Y.-R., Song,S.-D. and Gao,F.
TITLE Direct Submission
JOURNAL Submitted (06-APR-2012) Department of Physics, Tianjin University, No.92, Weijin Road, Nankai District, Tianjin 300072, China

Reference sequence for *Escherichia coli,* str. K-12 substr. DH10B:
LOCUS CP000948 4686137 bp DNA circular BCT 05-JUN-2008
DEFINITION Escherichia coli str. K12 substr. DH10B, complete genome.
ACCESSION CP000948
VERSION CP000948.1 GI:169887498
DBLINK BioProject: PRJNA20079 KEYWORDS .
SOURCE Escherichia coli str. K-12 substr. DH10B ORGANISM Escherichia coli str. K-12 substr. DH10B Bacteria; Proteobacteria; Gammaproteobacteria;
Enterobacteriales; Enterobacteriaceae; Escherichia.

### REFERENCE 1 (bases 1 to 4686137)

AUTHORS Durfee,T., Nelson,R., Baldwin,S., Plunkett,G. III, Burland,V., Mau,B., Petrosino,J.F., Qin,X., Muzny,D.M., Ayele,M., Gibbs,R.A., Csorgo,B., Posfai,G., The inventorsinstock,G.M. and Blattner,F.R.
TITLE The complete genome sequence of Escherichia coli DH10B: insights into the biology of a laboratory workhorse
JOURNAL J. Bacteriol. 190 (7), 2597-2606 (2008) PUBMED 18245285

### REFERENCE 2 (bases 1 to 4686137)

AUTHORS Plunkett,G. III.
TITLE Direct Submission
JOURNAL Submitted (20-FEB-2008) Department of Genetics and Biotechnology, University of Wisconsin, 425G Henry Mall, Madison, WI 53706, USA
COMMENT DH10B and DH10B-T1R are available from Invitrogen Corporation
   (http://www.invitrogen.com).

Reference sequence for *Klebsiella oxytoca,* strain NC_016612 (http://www.genome.jp/dbget-bin/www_bget?refseq+NC_016612): LOCUS NC_016612 5974109 bp DNA circular CON 07-FEB-2015
DEFINITION Klebsiella oxytoca KCTC 1686, complete genome.
ACCESSION NC_016612
VERSION NC_016612.1 GI:375256816
DBLINK BioProject: PRJNA224116 BioSample: SAMN02603580 Assembly: GCF_000240325.1
KEYWORDS RefSeq.
SOURCE Klebsiella oxytoca KCTC 1686 ORGANISM Klebsiella oxytoca KCTC 1686 Bacteria; Proteobacteria; Gammaproteobacteria;
Enterobacteriales; Enterobacteriaceae; Klebsiella.

### REFERENCE 1 (bases 1 to 5974109)

AUTHORS Shin,S.H., Kim,S., Kim,J.Y., Lee,S., Um,Y., Oh,M.K., Kim,Y.R., Lee,J. and Yang,K.S.
TITLE Complete genome sequence of Klebsiella oxytoca KCTC 1686, used in production of 2,3-butanediol
JOURNAL J. Bacteriol. 194 (9), 2371-2372 (2012) PUBMED 22493189

### REFERENCE 2 (bases 1 to 5974109)

AUTHORS Shin,S.H., Kim,S., Kim,J.Y., Yang,K.-S. and Seo,J.-S.
TITLE Direct Submission
JOURNAL Submitted (21-DEC-2011) Life Science Institute, Macrogen Inc., 10F, World Meridian Center, 60-24, Gasan-dong, Kumchun-gu, Seoul 153-781, Republic of Korea

Reference sequence for *Klebsiella pneumoniae,* strain NC_009648 (http://www.genome.jp/dbget-bin/www_bget?refseq+NC_009648): LOCUS NC_009648 5315120 bp DNA circular CON 07-FEB-2015
DEFINITION Klebsiella pneumoniae subsp. pneumoniae MGH 78578, complete sequence.
ACCESSION NC_009648
VERSION NC_009648.1 GI:152968582
DBLINK BioProject: PRJNA224116 BioSample: SAMN02603941 Assembly: GCF_000016305.1
KEYWORDS RefSeq.
SOURCE Klebsiella pneumoniae subsp. pneumoniae MGH 78578 ORGANISM Klebsiella pneumoniae subsp. pneumoniae MGH 78578 Bacteria; Proteobacteria; Gammaproteobacteria;
Enterobacteriales; Enterobacteriaceae; Klebsiella.

### REFERENCE 1 (bases 1 to 5315120)

AUTHORS McClelland,M., Sanderson,E.K., Spieth,J., Clifton,W.S., Latreille,P., Sabo,A., Pepin,K., Bhonagiri,V., Porwollik,S., Ali,J. and Wilson,R.K.
CONSRTM The Klebsiella pneumonia Genome Sequencing Project
TITLE Direct Submission
JOURNAL Submitted (06-SEP-2006) Genetics, Genome Sequencing Center, 4444 Forest Park Parkway, St. Louis, MO 63108, USA

Reference sequence for *Proteus mirabilis,* strain NC_010554 (http://www.genome.jp/dbget-bin/www_bget?refseq+NC_010554) : LOCUS NC_010554 4063606 bp DNA circular CON 07-FEB-2015
DEFINITION Proteus mirabilis strain HI4320, complete genome.
ACCESSION NC_010554
VERSION NC_010554.1 GI:197283915
DBLINK BioProject: PRJNA224116 Assembly: GCF_000069965.1
KEYWORDS RefSeq; complete genome.
SOURCE Proteus mirabilis HI4320
ORGANISM Proteus mirabilis HI4320 Bacteria; Proteobacteria; Gammaproteobacteria;
Enterobacteriales; Enterobacteriaceae; Proteus.

### REFERENCE 1

AUTHORS Pearson,M.M., Sebaihia,M., Churcher,C., Quail,M.A., Seshasayee,A.S., Luscombe,N.M., Abdellah,Z., Arrosmith,C., Atkin,B., Chillingworth,T., Hauser,H., Jagels,K., Moule,S., Mungall,K., Norbertczak,H., Rabbinowitsch,E., Walker,D., Whithead,S., Thomson,N.R., Rather,P.N., Parkhill,J. and Mobley,H.L.
TITLE Complete genome sequence of uropathogenic Proteus mirabilis, a master of both adherence and motility
JOURNAL J. Bacteriol. 190 (11), 4027-4037 (2008) PUBMED 18375554

### REFERENCE 2 (bases 1 to 4063606)

AUTHORS Sebaihia,M.
TITLE Direct Submission
JOURNAL Submitted (18-FEB-2008) Sebaihia M., Sulston Laboratories, Wellcome Trust Sanger Institute, Wellcome Trust Genome Campus, Hinxton, Cambridge, CB10 1SA, UNITED KINGDOM

Reference sequence for *Serratia marcescens,* strain NC_020211 (http://www.genome.jp/dbget-bin/www_bget?refseq+NC_020211) : LOCUS NC_020211 5241455 bp DNA circular CON 07-FEB-2015
DEFINITION Serratia marcescens WW4, complete genome.
ACCESSION NC_020211
VERSION NC_020211.1 GI:448239774
DBLINK BioProject: PRJNA224116 BioSample: SAMN02602965 Assembly: GCF_000336425.1
KEYWORDS RefSeq.
SOURCE Serratia marcescens WW4 ORGANISM Serratia marcescens WW4
   Bacteria; Proteobacteria; Gammaproteobacteria; Enterobacteriales; Enterobacteriaceae; Serratia.

### REFERENCE 1 (bases 1 to 5241455)

AUTHORS Kuo,P.A., Kuo,C.H., Lai,Y.K., Graumann,P.L. and Tu,J.
TITLE Phosphate limitation induces the intergeneric inhibition of Pseudomonas aeruginosa by Serratia marcescens isolated from paper machines
JOURNAL FEMS Microbiol. Ecol. 84 (3), 577-587 (2013) PUBMED 23398522

### REFERENCE 2 (bases 1 to 5241455)

AUTHORS Chung,W.C., Chen,L.L., Lo,W.S., Kuo,P.A., Tu,J. and Kuo,C.H.
TITLE Complete Genome Sequence of Serratia marcescens WW4
JOURNAL Genome Announc 1 (2), E0012613 (2013) PUBMED 23558532
REMARK Publication Status: Online-Only

### REFERENCE 3 (bases 1 to 5241455)

AUTHORS Chung,W.-C., Chen,L.-L., Lo,W.-S., Kuo,P.-A., Tu,J. and Kuo,C.-H.
TITLE Direct Submission
JOURNAL Submitted (26-NOV-2012) Institute of Plant and Microbial Biology, Academia Sinica, 128 Sec. 2, Academia Rd., Taipei 115, Taiwan

Raw paired-end sequencing data for the samples were mapped also against the respective pan-genomes with BWA 0.6.1.20, as above. The resulting SAM files were sorted, converted to BAM files, and PCR duplicates were marked using the Picard tools package 1.104 (http://picard.sourceforge.net/).

### Data analysis

For the chromosomal model using the reference genomes, analysis was as follows: The Genome Analysis Toolkit 3.1.1 (GATK) was used to call SNPs and indels for blocks of 200 samples (parameters: - ploidy 1 -glm BOTH -stand_call_conf 30 -stand_emit_conf 10). VCF files were combined into a single file and quality filtering for SNPs was carried out (QD < 2.0 || FS > 60.0 || MQ < 40.0) and indels (QD < 2.0 || FS > 200.0). Detected variants were annotated with SnpEff22 to predict coding effects.

For matching the obtained SNPs in the reference genomes with the pan-genomes, the following analysis was carried out:
1. The gene containing the variant was identified (gene entry in the corresponding GenBank file of the reference genome), i.e. the genomic position of the variant must be within the gene start and end interval. The start and end positions of the subsequence containing the variant were determined as follows: Start was set to (genomic positon of the variant - 250 bases) and end to (genomic position of the variant + 250 bases). If the start/end position were beyond the gene start/end then they were set to start/end positions of the gene.
2. The extracted variant containing subsequences were aligned against the pan-genome of the corresponding species using blastn.
3. The blastn results were filtered: Only matches with sequence identity >= 80% and aligning >= 80% of the variant containing subsequence were kept.
4. For each of the remaining matches the position of the variant within the pan-genome gene was determined.
5. These positions were searched in the corresponding filtered VCF files (i.e. VCF (Variant Call Format) file containing analyzed variants). For easier analysis, only the 50 SNPs with the best p-values in the SNP analysis were taken into account. Only variants which could be mapped to the pan-genome and were found in corresponding VCF files were considered: They were coded as binary variables: 0 = no reference allele, 1 = reference allele, NA = missing.

Resistance profiles were determined with w.r.t. EUCAST MIC breakpoint guidelines (v. 4). Samples considered were only samples after filtering w.r.t. assembly quality and taxonomic assignment. For obtaining an optimized model, decision tree analysis was carried out as follows:
Model: Decision tree from R-package rpart; maximal depth=5, other parameters set to "force" more complex trees containing multiple features; the set parameters affect the number of features in the model

Selected results for the 6 different species are given in tables 2 to 7. In the tables, the column "drug" refers to the respective antibiotic used in each exemplary analysis, columns 2 and 3 refer to the model using only chromosomal nucleic acid sequences with the reference genome, and columns 4 and 5 refers to the model obtained using the pan-genome (that take into account both chromosomal and plasmid nucleic acid sequences) (termed "PG model"). Further, the term "B_ACC" refers to the balanced accuracy, and the term "features" refers to the different SNPs that were used in the decision trees obtained for the respective models in tables 2 to 7.

The balanced accuracy was thereby used for analyzing the data as it gives more balanced results, as explained in the following.

The balanced accuracy is defined as the arithmetic mean of sensitivity and specificity = (sensitivity + specificity)/2 with sensitivity = TP / (TP+FN) and specificity = TN / (TN+FP).
- TN = true negatives = susceptible and predicted to be susceptible
- TP = true positives = resistant and predicted to be resistant
- FN = false negatives = resistance, predicted to be susceptible
- FP = false positives = susceptible, predicted to be resistance

It is a better performance estimate than accuracy ( (TP + TN) / (number of samples)) in case of imbalanced datasets, e.g. if there are much more resistant samples when non-resistant ones or vice versa. In such cases accuracy may be high, although the "smaller" class is not predicted correctly, as seen in the following exemplary numerical case - the balanced accuracy is less biased by the data imbalance.

Exemplary numerical case: 11 samples are resistant, 51 are susceptible and TP=50, TN=1, FN=1, FP=10. Then accuracy = (50+1) / 62 = 82.26% and balanced accuracy is (( 50/51 ) + ( 1/11 ))/2 = 53.57%.

A comparison of the two models is only shown in the subsequent tables if both models have at least 2 unique variants.

**Table 2: Results of Examples for Acinetobacter baumannii**

| Drug | B_ACC using only chromosome | number of features (chromosome) | B_ACC (PG model) | PG model number of features |
|---|---|---|---|---|
| CP | 80.7 | 4 | 85.34 | 7 |
| IMP | 61.2 | 3 | 62.11 | 2 |
| LVX | 89.35 | 7 | 91.48 | 3 |

**Table 3: Results of Examples for Escherichia coli**

| Drug | B_ACC using only chromosome | number of features (chromosome) | B_ACC (PG model) | PG model number of features |
|---|---|---|---|---|
| CRM | 55.65 | 8 | 62.71 | 7 |

**Table 4: Results of Examples for Klebsiella oxytoca**

| Drug | B_ACC using only chromosome | number of features (chromosome) | B_ACC (PG model) | PG model number of features |
|---|---|---|---|---|
| CP | 93.05 | 3 | 93.76 | 2 |

**Table 5: Results of Examples for Klebsiella pneumoniae**

| Drug | B_ACC using only chromosome | number of features (chromosome) | B_ACC (PG model) | PG model number of features |
|---|---|---|---|---|
| A/S | 72.6 | 5 | 77.48 | 4 |
| AZT | 88.5 | 6 | 88.81 | 2 |
| CAZ | 87.65 | 6 | 87.98 | 4 |
| CRM | 79.65 | 7 | 81.83 | 4 |
| GM | 70.45 | 8 | 70.47 | 12 |

**Table 6: Results of Examples for Proteus mirabilis**

| Drug | B_ACC using only chromosome | number of features (chromosome) | B_ACC (PG model) | PG model number of features |
|---|---|---|---|---|
| AM | 70.75 | 2 | 74.46 | 4 |
| A/S | 51.3 | 2 | 62.91 | 2 |
| CP | 86.35 | 2 | 89.11 | 5 |
| LVX | 83.75 | 2 | 87.77 | 4 |
| T/S | 68.45 | 2 | 71.35 | 3 |

**Table 7: Results of Examples for Serratia marcescens**

| Drug | B_ACC using only chromosome | number of features (chromosome) | B_ACC (PG model) | PG model number of features |
|---|---|---|---|---|
| AZT | 58.7 | 8 | 69.67 | 5 |
| CAX | 58.1 | 5 | 68.08 | 2 |
| CAZ | 60.05 | 3 | 62.82 | 5 |
| CFT | 58.6 | 6 | 67.45 | 5 |

As can be seen from the tables, the balanced accuracy - and therefore the prediction of antibiotic resistance - improved for the different tested drugs all over the different bacterial species using the pan-genome approach.

It is noted that the number of features in the tables for achieving the best results in balanced accuracy often vary, which is a result of the optimization approach which was used in the present models for achieving these results and which were not limited to achieve certain comparable numbers of features in the decision trees. Notably, though, the variances in numbers of features shifts in both directions, i.e. sometimes more feature numbers are obtained in the model using chromosomal nucleic acid sequences only, and sometimes more feature numbers are obtained in the PG model, showing that there is no bias in any of the models for a higher number of features. Further, the data for the drug A/S for Proteus mirabilis actually had the same number of features for both models, which show that the effect is also independent of the number of features and can be obtained for a combination of at least two features. It is to be noted that the features in the two models in this case also differed. Overall an improvement in performance is seen when the pan-genome is used for analysis.

Further to the results in the tables, it was also again confirmed that a combination of more than one, e.g. i (being a natural number), variants performed significantly better than single variants. It was also seen in the data that the power of predicting genetic resistance increased if variants from different genes were selected.

While in the first step all i variants could have been theoretically selected from the same gene, the performance increased when j (again being a natural number) variants are selected that come from at least two different genes. Further improved results were obtained in the pan-genome model, as seen in the above tables.

In the examples, it was demonstrated that the performance of predicting resistance of the bacteria further increases if the full set of all genes in a pan-genome is used. By using the full genetic set available, we outperformed the results that are solely based on the bacterial chromosome.

As already stated above, notably the information described herein does not only refer to single variants (SNPs) and combinations of those. The same also applies for the above-mentioned larger structural variations; also in this case the performance gets better if genes are analyzed using pan-genomes in the bioinformatics analysis as compared to the performance when using a reference genome from a single isolate.

## Claims

1. A data bank comprising at least one pan-genome of one of the following bacterial species:
- *Escherichia coli,*
- *Acinetobacter baumanii,*
- *Citrobacter koseri,*
- *Enterobacter aerogenes,*
- *Enterobacter cloacae,*
- *Klebsiella oxytoca,*
- *Klebsiella pneumoniae,*
- *Morganella morganii,*
- *Proteus mirabilis,*
- *Pseudomonas aeruginosa,*
- *Salmonella enterica,*
- *Staphylococcus aureus,*
- *Serratia marcescens,*
- *Shigella boydii,*
- *Shigella flexneri,*
- *Shigella sonnei,* and
- *Stenotrophomonas maltophilia,*
wherein the pan-genome of *Escherichia coli* comprises or consists of SEQ ID NO 1 - 41296 or comprises or consists of sequences that are at least 90% identical to SEQ ID NO 1 - 41296 or comprises at least 90% of SEQ ID NO 1 - 41296, the pan-genome of *Acinetobacter baumanii* comprises or consists of SEQ ID NO 41297 - 71315 or comprises or consists of sequences that are at least 90% identical to SEQ ID NO 41297 - 71315 or comprises at least 90% of SEQ ID NO 41297 - 71315, the pan-genome of *Citrobacter koseri* comprises or consists of SEQ ID NO 71316 - 86840 or comprises or consists of sequences that are at least 90% identical to SEQ ID NO 71316 - 86840 or comprises at least 90% of SEQ ID NO 71316 - 86840, the pan-genome of *Enterobacter aerogenes* comprises or consists of SEQ ID NO 86841 - 113784 or comprises or consists of sequences that are at least 90% identical to SEQ ID NO 86841 - 113784 or comprises at least 90% of SEQ ID NO 86841 - 113784, the pan-genome of *Enterobacter cloacae* comprises or consists of SEQ ID NO 113785 - 171921 or comprises or consists of sequences that are at least 90% identical to SEQ ID NO 113785 - 171921 or comprises at least 90% of SEQ ID NO 113785 - 171921, the pan-genome of *Klebsiella oxytoca* comprises or consists of SEQ ID NO 171922 - 209528 or comprises or consists of sequences that are at least 90% identical to SEQ ID NO 171922 - 209528 or comprises at least 90% of SEQ ID NO 171922 - 209528, the pan-genome of *Klebsiella pneumoniae* comprises or consists of SEQ ID NO 209529 - 273591 or comprises or consists of sequences that are at least 90% identical to SEQ ID NO 209529 - 273591 or comprises at least 90% of SEQ ID NO 209529 - 273591, the pan-genome of *Morganella morganii* comprises or consists of SEQ ID NO 273592 - 293457 or comprises or consists of sequences that are at least 90% identical to SEQ ID NO 273592 - 293457 or comprises at least 90% of SEQ ID NO 273592 - 293457, the pan-genome of *Proteus mirabilis* comprises or consists of SEQ ID NO 293458 - 318000 or comprises or consists of sequences that are at least 90% identical to SEQ ID NO 293458 - 318000 or comprises at least 90% of SEQ ID NO 293458 - 318000, the pan-genome of *Pseudomonas aeruginosa* comprises or consists of SEQ ID NO 318001 - 368053 or comprises or consists of sequences that are at least 90% identical to SEQ ID NO 318001 - 368053 or comprises at least 90% of SEQ ID NO 318001 - 368053, the pan-genome of *Salmonella enterica* comprises or consists of SEQ ID NO 368054 - 404774 or comprises or consists of sequences that are at least 90% identical to SEQ ID NO 368054 - 404774 or comprises at least 90% of SEQ ID NO 368054 - 404774, the pan-genome of *Staphylococcus aureus* comprises or consists of SEQ ID NO 404775 - 421990 or comprises or consists of sequences that are at least 90% identical to SEQ ID NO 404775 - 421990 or comprises at least 90% of SEQ ID NO 404775 - 421990, the pan-genome of *Serratia marcescens* comprises or consists of SEQ ID NO 421991 - 457642 or comprises or consists of sequences that are at least 90% identical to SEQ ID NO 421991 - 457642 or comprises at least 90% of SEQ ID NO 421991 - 457642, the pan-genome of *Shigella boydii* comprises or consists of SEQ ID NO 457643 - 470165 or comprises or consists of sequences that are at least 90% identical to SEQ ID NO 457643 - 470165 or comprises at least 90% of SEQ ID NO 457643 - 470165, the pan-genome of *Shigella flexneri* comprises or consists of SEQ ID NO 470166 - 481587 or comprises or consists of sequences that are at least 90% identical to SEQ ID NO 470166 - 481587 or comprises at least 90% of SEQ ID NO 470166 - 481587, the pan-genome of *Shigella sonnei* comprises or consists of SEQ ID NO 481588 - 492644 or comprises or consists of sequences that are at least 90% identical to SEQ ID NO 481588 - 492644 or comprises at least 90% of SEQ ID NO 481588 - 492644, and the pan-genome of *Stenotrophomonas maltophilia* comprises or consists of SEQ ID NO 492645 - 537429 or comprises or consists of sequences that are at least 90% identical to SEQ ID NO 492645 - 537429 or comprises at least 90% of SEQ ID NO 492645 - 537429.

2. The data bank of claim 1, wherein the data bank comprises 2, 3, 4, 5, 6, 7, 8, 9, 10, 11, 12, 13, 14, 15, 16, or 17 of the pan-genomes.

3. Use of the data bank of claim 1 or 2 for the determination of bacterial resistance in a sample from a patient.

4. A pan-genome of one of the following bacterial species:
- *Escherichia coli,*
- *Acinetobacter baumanii,*
- *Citrobacter koseri,*
- *Enterobacter aerogenes,*
- *Enterobacter cloacae,*
- *Klebsiella oxytoca,*
- *Klebsiella pneumoniae,*
- *Morganella morganii,*
- *Proteus mirabilis,*
- *Pseudomonas aeruginosa,*
- *Salmonella enterica,*
- *Staphylococcus aureus,*
- *Serratia marcescens,*
- *Shigella boydii,*
- *Shigella flexneri,*
- *Shigella sonnei,* and
- *Stenotrophomonas maltophilia,*
wherein the pan-genome of *Escherichia coli* comprises or consists of SEQ ID NO 1 - 41296 or comprises or consists of sequences that are at least 90% identical to SEQ ID NO 1 - 41296 or comprises at least 90% of SEQ ID NO 1 - 41296, the pan-genome of *Acinetobacter baumanii* comprises or consists of SEQ ID NO 41297 - 71315 or comprises or consists of sequences that are at least 90% identical to SEQ ID NO 41297 - 71315 or comprises at least 90% of SEQ ID NO 41297 - 71315, the pan-genome of *Citrobacter koseri* comprises or consists of SEQ ID NO 71316 - 86840 or comprises or consists of sequences that are at least 90% identical to SEQ ID NO 71316 - 86840 or comprises at least 90% of SEQ ID NO 71316 - 86840, the pan-genome of *Enterobacter aerogenes* comprises or consists of SEQ ID NO 86841 - 113784 or comprises or consists of sequences that are at least 90% identical to SEQ ID NO 86841 - 113784 or comprises at least 90% of SEQ ID NO 86841 - 113784, the pan-genome of *Enterobacter cloacae* comprises or consists of SEQ ID NO 113785 - 171921 or comprises or consists of sequences that are at least 90% identical to SEQ ID NO 113785 - 171921 or comprises at least 90% of SEQ ID NO 113785 - 171921, the pan-genome of *Klebsiella oxytoca* comprises or consists of SEQ ID NO 171922 - 209528 or comprises or consists of sequences that are at least 90% identical to SEQ ID NO 171922 - 209528 or comprises at least 90% of SEQ ID NO 171922 - 209528, the pan-genome of *Klebsiella pneumoniae* comprises or consists of SEQ ID NO 209529 - 273591 or comprises or consists of sequences that are at least 90% identical to SEQ ID NO 209529 - 273591 or comprises at least 90% of SEQ ID NO 209529 - 273591, the pan-genome of *Morganella morganii* comprises or consists of SEQ ID NO 273592 - 293457 or comprises or consists of sequences that are at least 90% identical to SEQ ID NO 273592 - 293457 or comprises at least 90% of SEQ ID NO 273592 - 293457, the pan-genome of *Proteus mirabilis* comprises or consists of SEQ ID NO 293458 - 318000 or comprises or consists of sequences that are at least 90% identical to SEQ ID NO 293458 - 318000 or comprises at least 90% of SEQ ID NO 293458 - 318000, the pan-genome of *Pseudomonas aeruginosa* comprises or consists of SEQ ID NO 318001 - 368053 or comprises or consists of sequences that are at least 90% identical to SEQ ID NO 318001 - 368053 or comprises at least 90% of SEQ ID NO 318001 - 368053, the pan-genome of *Salmonella enterica* comprises or consists of SEQ ID NO 368054 - 404774 or comprises or consists of sequences that are at least 90% identical to SEQ ID NO 368054 - 404774 or comprises at least 90% of SEQ ID NO 368054 - 404774, the pan-genome of *Staphylococcus aureus* comprises or consists of SEQ ID NO 404775 - 421990 or comprises or consists of sequences that are at least 90% identical to SEQ ID NO 404775 - 421990 or comprises at least 90% of SEQ ID NO 404775 - 421990, the pan-genome of *Serratia marcescens* comprises or consists of SEQ ID NO 421991 - 457642 or comprises or consists of sequences that are at least 90% identical to SEQ ID NO 421991 - 457642 or comprises at least 90% of SEQ ID NO 421991 - 457642, the pan-genome of *Shigella boydii* comprises or consists of SEQ ID NO 457643 - 470165 or comprises or consists of sequences that are at least 90% identical to SEQ ID NO 457643 - 470165 or comprises at least 90% of SEQ ID NO 457643 - 470165, the pan-genome of *Shigella flexneri* comprises or consists of SEQ ID NO 470166 - 481587 or comprises or consists of sequences that are at least 90% identical to SEQ ID NO 470166 - 481587 or comprises at least 90% of SEQ ID NO 470166 - 481587, the pan-genome of *Shigella sonnei* comprises or consists of SEQ ID NO 481588 - 492644 or comprises or consists of sequences that are at least 90% identical to SEQ ID NO 481588 - 492644 or comprises at least 90% of SEQ ID NO 481588 - 492644, and the pan-genome of *Stenotrophomonas maltophilia* comprises or consists of SEQ ID NO 492645 - 537429 or comprises or consists of sequences that are at least 90% identical to SEQ ID NO 492645 - 537429 or comprises at least 90% of SEQ ID NO 492645 - 537429.

5. A method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations by comparison to a first pan-genome to obtain a third data set of genetic variants;
providing a second data set of antimicrobial drug resistance and/or susceptibility of the at least one clinical isolate of the microorganism;
correlating the third data set with the second data set and statistically analyzing the correlation;
determining the genetic variations in the at least one clinical isolate of the microorganism associated with antimicrobial drug resistance; and
determining a second pan-genome using the first pan-genome as well as the genomes used for constructing it and the first data set of at least one nucleic acid sequence of the at least one clinical isolate of the microorganism.

6. A method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations correlated to antimicrobial resistance and/or susceptibility by comparison to a first pan-genome, wherein the first pan-genome is obtained at least partially using a plurality of clinical isolates of the microorganism for which genetic variations are correlated to antimicrobial resistance and/or susceptibility;
determining the genetic variations in the at least one clinical isolate of the microorganism associated with antimicrobial drug resistance; and
determining a second pan-genome using the first pan-genome as well as the genomes used for constructing it and the first data set of at least one nucleic acid sequence of the at least one clinical isolate of the microorganism.

7. A method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations correlated to antimicrobial resistance and/or susceptibility by comparison to a first pan-genome, wherein the first pan-genome is obtained at least partially using a plurality of clinical isolates of the microorganism for which genetic variations are correlated to antimicrobial resistance and/or susceptibility; and
determining the genetic variations in the at least one clinical isolate of the microorganism associated with antimicrobial drug resistance.

8. A method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations not correlated to antimicrobial resistance and/or susceptibility by comparison to a first pan-genome to obtain a third data set of genetic variants, and analyzing the at least one nucleic acid sequence of the first data set for genetic variations correlated to antimicrobial resistance and/or susceptibility by comparison to the first pan-genome, wherein the first pan-genome is obtained at least partially using a plurality of clinical isolates of the microorganism for which genetic variations are correlated to antimicrobial resistance and/or susceptibility;
providing a second data set of antimicrobial drug resistance and/or susceptibility of the at least one clinical isolate of the microorganism;
correlating the third data set with the second data set and statistically analyzing the correlation;
determining the genetic variations in the at least one clinical isolate of the microorganism associated with antimicrobial drug resistance; and
determining a second pan-genome using the first pan-genome as well as the genomes used for constructing it and the first data set of at least one nucleic acid sequence of the at least one clinical isolate of the microorganism.

9. A method of determining an antimicrobial drug resistance profile for at least one clinical isolate of a microorganism and for improving a first pan-genome, comprising:
obtaining or providing a first data set of at least one nucleic acid sequence of the at least one clinical isolate of the microorganism, wherein optionally at least a part of the nucleic acid sequence of the first data set is assembled;
analyzing the at least one nucleic acid sequence of the first data set for genetic variations not correlated to antimicrobial resistance and/or susceptibility by comparison to a first pan-genome to obtain a third data set of genetic variants, and analyzing the at least one nucleic acid sequence of the first data set for genetic variations correlated to antimicrobial resistance and/or susceptibility by comparison to the first pan-genome, wherein the first pan-genome is obtained at least partially using a plurality of clinical isolates of the microorganism for which genetic variations are correlated to antimicrobial resistance and/or susceptibility;
providing a second data set of antimicrobial drug resistance and/or susceptibility of the at least one clinical isolate of the microorganism;
correlating the third data set with the second data set and statistically analyzing the correlation; and
determining the genetic variations in the at least one clinical isolate of the microorganism associated with antimicrobial drug resistance.

10. The method of any one of claims 5 to 9, wherein the first pan-genome is a pan-genome of claim 4.

11. The method of any one of claims 5 to 9, wherein
the first pan-genome is for *Escherichia coli* and is obtained using at least 150 clinical isolates, preferably at least 250 clinical isolates; or wherein
the first pan-genome is for *Acinetobacter baumanii* and is obtained using at least 150 clinical isolates, preferably at least 180 clinical isolates; or wherein
the first pan-genome is for *Citrobacter koseri* and is obtained using at least 35 clinical isolates, preferably at least 55 clinical isolates; or wherein
the first pan-genome is for *Enterobacter aerogenes* and is obtained using at least 120 clinical isolates, preferably at least 160 clinical isolates; or wherein
the first pan-genome is for *Enterobacter cloacae* and is obtained using at least 100 clinical isolates, preferably at least 150 clinical isolates; or wherein
the first pan-genome is for *Klebsiella oxytoca* and is obtained using at least 90 clinical isolates, preferably at least 120 clinical isolates; or wherein
the first pan-genome is for *Klebsiella pneumoniae* and is obtained using at least 160 clinical isolates, preferably at least 250 clinical isolates; or wherein
the first pan-genome is for *Morganella morganii* and is obtained using at least 70 clinical isolates, preferably at least 120 clinical isolates; or wherein
the first pan-genome is for *Proteus mirabilis* and is obtained using at least 120 clinical isolates, preferably at least 140 clinical isolates; or wherein
the first pan-genome is for *Pseudomonas aeruginosa* and is obtained using at least 100 clinical isolates, preferably at least 140 clinical isolates; or wherein
the first pan-genome is for *Salmonella enterica* and is obtained using at least 130 clinical isolates, preferably at least 190 clinical isolates; or wherein
the first pan-genome is for *Staphylococcus aureus* and is obtained using at least 180 clinical isolates, preferably at least 260 clinical isolates; or wherein
the first pan-genome is for *Serratia marcescens* and is obtained using at least 230 clinical isolates, preferably at least 330 clinical isolates; or wherein
the first pan-genome is for *Shigella boydii* and is obtained using at least 60 clinical isolates, preferably at least 75 clinical isolates; or wherein
the first pan-genome is for *Shigella flexneri* and is obtained using at least 50 clinical isolates, preferably at least 80 clinical isolates; or wherein
the first pan-genome is for *Shigella sonnei* and is obtained using at least 60 clinical isolates, preferably at least 80 clinical isolates; or wherein
the first pan-genome is for *Stenotrophomonas maltophilia* and is obtained using at least 125 clinical isolates, preferably at least 240 clinical isolates.

12. A pan-genome, wherein
the pan-genome is for *Escherichia coli* and is obtained using at least 150 clinical isolates, preferably at least 250 clinical isolates; or wherein
the pan-genome is for *Acinetobacter baumanii* and is obtained using at least 150 clinical isolates, preferably at least 180 clinical isolates; or wherein
the pan-genome is for *Citrobacter koseri* and is obtained using at least 35 clinical isolates, preferably at least 55 clinical isolates; or wherein
the pan-genome is for *Enterobacter aerogenes* and is obtained using at least 120 clinical isolates, preferably at least 160 clinical isolates; or wherein
the pan-genome is for *Enterobacter cloacae* and is obtained using at least 100 clinical isolates, preferably at least 150 clinical isolates; or wherein
the pan-genome is for *Klebsiella oxytoca* and is obtained using at least 90 clinical isolates, preferably at least 120 clinical isolates; or wherein
the pan-genome is for *Klebsiella pneumoniae* and is obtained using at least 160 clinical isolates, preferably at least 250 clinical isolates; or wherein
the pan-genome is for *Morganella morganii* and is obtained using at least 70 clinical isolates, preferably at least 120 clinical isolates; or wherein
the pan-genome is for *Proteus mirabilis* and is obtained using at least 120 clinical isolates, preferably at least 140 clinical isolates; or wherein
the pan-genome is for *Pseudomonas aeruginosa* and is obtained using at least 100 clinical isolates, preferably at least 140 clinical isolates; or wherein
the pan-genome is for *Salmonella enterica* and is obtained using at least 130 clinical isolates, preferably at least 190 clinical isolates; or wherein
the pan-genome is for *Staphylococcus aureus* and is obtained using at least 180 clinical isolates, preferably at least 260 clinical isolates; or wherein
the pan-genome is for *Serratia marcescens* and is obtained using at least 230 clinical isolates, preferably at least 330 clinical isolates; or wherein
the pan-genome is for *Shigella boydii* and is obtained using at least 60 clinical isolates, preferably at least 75 clinical isolates; or wherein
the pan-genome is for *Shigella flexneri* and is obtained using at least 50 clinical isolates, preferably at least 80 clinical isolates; or wherein
the pan-genome is for *Shigella sonnei* and is obtained using at least 60 clinical isolates, preferably at least 80 clinical isolates; or wherein
the pan-genome is for *Stenotrophomonas maltophilia* and is obtained using at least 125 clinical isolates, preferably at least 240 clinical isolates.

13. Use of the pan-genome of claim 4 or 12 for the determination of bacterial resistance in a sample from a patient.

14. A method of determining an infection of a patient with a microorganism, comprising the steps of:
a) obtaining or providing a sample containing or suspected of containing a microorganism from the patient;
b) determining the presence of at least one genetic variation in at least one genetic sequence of the microorganism, as determined by the method of any one of claims 5 to 11,
wherein the presence of said at least one genetic variation is indicative of an infection with an antimicrobial drug resistant microorganism in said patient.

15. The method of claim 14, wherein the microorganism is a bacterial microorganism potentially resistant to antimicrobial drug treatment.

16. A method of selecting a treatment of a patient suffering from an infection with a potentially resistant microorganism, comprising the steps of:
a) obtaining or providing a sample containing or suspected of containing a microorganism from the patient;
b) determining the presence of at least one genetic variation in at least one genetic sequence of the microorganism, as determined by the method of any one of claims 5 to 11, wherein the presence of said at least one genetic variation is indicative of an infection with an antimicrobial drug resistant microorganism in said patient;
c) identifying said at least one or more antimicrobial drugs; and
d) selecting one or more antimicrobial drugs different from the ones identified in step c) and being suitable for the treatment of the infection with the microorganism.

17. Computer program product comprising computer executable instructions which, when executed, perform a method according to any one of claims 5 to 11 and 14 to 16.
